# EUROPEAN PATENT SPECIFICATION

(11) **EP 4 360 592 B1**
(45) Date of publication and mention of the grant of the patent: **13.05.2026**
(21) Application number: 22203586.7
(22) Date of filing: 25.10.2022
(51) Int. Cl.: A61F 2/24, A61F 2/00

(54) **A CARDIAC AORTO-MITRAL PROSTHESIS FOR THE HUMAN HEART**
HERZAORTO-MITRALPROTHESE FÜR DAS MENSCHLICHE HERZ
PROTHÈSE AORTO-MITRALE CARDIAQUE POUR LE COEUR HUMAIN

(43) Date of publication of application: 01.05.2024
(73) Proprietor: Andrasi, Terezia, 35037 Marburg (DE)
(72) Inventor: Andrasi, Terezia, 35037 Marburg (DE)
(74) Representative: Meyer-Dulheuer MD Legal Patentanwälte PartG mbB

(56) References cited:
- US-A1- 2011 230 810
- BASEL RAMLAWI ET AL: "Endocarditis with Involvement of the Aorto-Mitral Curtain", OPERATIVE TECHNIQUES IN THORACIC AND CARDIOVASCULAR SURGERY, vol. 16, no. 3, 25 October 2019 (2019-10-25), pages 242 - 249, XP028392709, ISSN: 1522-2942, [retrieved on 20111025], DOI: 10.1053/J.OPTECHSTCVS.2011.09.004

## Description

The invention is directed to a cardiac aorto-mitral prosthesis for the human heart that replaces the cardiac aorto-mitral curtain during surgical operations to the human heart, as defined by claim 1. The prosthesis is capable to anatomically replace the cardiac aorto-mitral curtain which may be affected during a replacement of the mitral valve or of the aortic valve. The cardiac aorto-mitral prosthesis offers a shaping that matches the anatomic shape of the intervalvular fibrous body including the left and right fibrous trigons between the mitral valve and the aortic valve. The cardiac aorto-mitral prosthesis can be implanted quickly during a surgical operation, thereby shortening the operation time and simplifying the surgical procedure. The cardiac aorto-mitral improves the recovery perspectives and the physical wellness of the patient after the surgical operation. The invention is also directed to a process for the production of the cardiac aorto-mitral prosthesis, as defined by claim 13.

Surgical operations to the human heart are nowadays frequently carried out for the anatomic replacement of the mitral valve, the aortic valve or both the mitral valve and the aortic valve. Such a replacement of the aortic valve or the mitral valve is often caused by a valve endocarditis. Between the aortic valve annulus and the mitral valve annulus, an intervalvular fibrous body is anatomically situated which is part of the central fibrous body of the human heart. During operations for the replacement of the aortic valve or the mitral valve, the tissue of the intervalvular fibrous body of the human heart between the aortic valve annulus and the mitral valve annulus must be replaced frequently. The reasons for such a replacement may be for instance a damage of the intervalvular fibrous body by an infective endocarditis or a degenerative calcification. The intervalvular fibrous body in the human heart in the specific position between the aortic valve annulus and the mitral valve annulus is also known in the art as the cardiac aorto-mitral curtain.

The replacement of the intervalvular fibrous body is known in the state of the art. The reconstructive technique that is currently most widely used for a replacement of the intervalvular fibrous body during an aortic and mitral valve replacement was described and pioneered by T. E. David et al. (T. E. David et al., J. Thorac. Cardiovasc. Surg., "Aortic and mitral valve replacement with reconstruction of the intervalvular fibrous body", volume 114 (5), pages 766-771, 1997). This method uses a single pericardial patch to reconstruct the intervalvular fibrous body by an artificial cardiac aorto-mitral curtain. After excising the aortic valve and the mitral valve, the diseased intervalvular fibrous body is removed and a properly tailored patch of Dacron-fabric or bovine pericardium is sutured to the lateral and medial fibrous trigones and the aortic root, reestablishing the annuli of the aortic and mitral valve. Then, the aortic valve and the mitral valve are replaced. This method reconstructs the cardiac aorto-mitral curtain for a replacement of the intervalvular fibrous body, but does not replace the anatomically right and left fibrous trigons of the human heart. The right fibrous trigon and left fibrous trigon of the heart are therefore often left insufficiently attended during an aortic valve or a mitral valve replacement.

The replacement of the intervalvular fibrous body in the surgery of the human heart is often called in the state of the art the "Commando"-operation.

The risks of the method as described by T. E. David et al. have been studied. Davierwala et al. (Davierwala et al., European Journal of Cardio-Thoracic Surgery "Double valve replacement and reconstruction of the intervalvular fibrous body in patients with active infective endocarditis", volume 45, issue 1, pages 146-152, 2014) further examined the risks that are involved with such a replacement of the intervalvular fibrous body during an infective endocarditis. Statistic data for the chances of success and for the mortality rates of the operation for the replacement of the intervalvular fibrous body were established. The same method as with T. E. David et al. was applied. A single pericardial patch was used to reconstruct the intervalvular fibrous body. This pericardial patch was an elliptical pericardial patch to which the anterior annular circumference of the mitral valve prosthesis and the non-coronary circumference of the aortic valve prosthesis were sutured. It was concluded that the reconstruction of the intervalvular fibrous body is an operation with a certain risk, but is the only option to cure a damage of the intervalvular fibrous body by infective endocarditis. This disease without treatment culminates into a 100 %-mortality.

Petterson et al. (Petterson et al., Multimed Man Cardiothorac Surg., "Reconstruction of fibrous skeleton: technique, pitfalls and results", 2014, DOI:10.1093/mmcts/mmu004) describe the reconstruction of a disrupted or destroyed intervalvular fibrous body during an aortic and mitral valve replacement. The importance of the sizing of the mitral valve and the use of an allograft for the aortic valve is described. The same method as in T. E. David et al. was applied. An elliptical pericardial patch was used to reconstruct the intervalvular fibrous body. The problems of a replacement of the right fibrous trigone and the left fibrous trigons of the human heart are not mentioned and are still left insufficiently attended.

Ramlawi et al. (Basel Ramlavi et al., Operative Techniques in Thoracic and Cardiovascular Surgery, "Endocarditis with Involvement of the Aorto-Mitral Curtain", Volume 16, No. 3, October 25, 2019, XP028392709, ISSN: 1522-2942, DOI: 10.1053/J.OPTECHSTCVS.2011.09.004, retrieved on October 25, 2011, pages 242-249) describe a surgical treatment of an endocarditis with the aortic valve and the mitral valve of the human heart wherein the aorto-mitral curtain is involved. The document gives a descriptive illustration of the anatomy of the human heart around the aorto-mitral valve complex and describes the removal of an infected aortic valve wherein destruction of the aorto-mitral curtain below the aortic annulus is revealed. This destruction is described to be typically a defect comprising infected or devitalized tissue in the aorto-mitral curtain below the aortic annulus which is then surgically resected and repaired with a patch using pericardium. The document does not show or describe any defect of tissue which involves or uncovers the trigones of the human heart.

In a number of operations for the replacement of the intervalvular fibrous body, however, the replacement of the entire intervalvular fibrous body located between the aortic valve annulus and the mitral valve annulus and including the entire left trigon and the entire right trigon is necessary. Especially when a larger area of the intervalvular fibrous body has been infected and the infection extends to the annuli of the aortic valve or the mitral valve or both, a larger area of the intervalvular fibrous body must be replaced. Whenever surgical excision of the fibrous trigons is implemented, the replacements as proposed above become insufficient, because the pericardial patch does not fit to the shape of the entire cardiac aorto-mitral curtain including the trigons. Removal of the trigons without replacement causes tension on the surgical sutures, and the surgical operations are less successful depending on how much tissue of the trigons is missing. Consequently, suture disruption and uncontrollable life-threatening bleeding may occur intraoperatively and considerable late complications related to massive hemorrhage, further infections, and development of interventricular or interatrial shunts and aneurysms may follow.

Often the right fibrous trigon located between the annulus of the tricuspid valve, the annulus of the aortic valve and the annulus of the mitral valve is affected by disruptions and tensions. To resolve this problem, an additional quadrangular pericardial patch may be used to replace the portion of the right fibrous trigon that is situated between the right coronary or the non-coronary commissure of the aortic valve and the additional segment of the aortic valve annulus up to the septal segment of the tricuspid valve annulus. This procedure may reduce the tensions on the right-sided suture of the pericardial patch replacing the cardiac aorto-mitral curtain, however, the left trigon, which is difficult to access, remains unattended and may give rise to postoperative suture disruptions and bleeding. Importantly, the left trigon remains unattended in all surgical procedures known up to date, and its anatomical position below and close to the origin of the left main coronary artery represents the most difficult localization to access during a surgical operation. As a result, the tension on the cardiac aorto-mitral curtain is increased which may give rise to tensions in the cardiac aorto-mitral curtain, a subsequent disruption of the cardiac aorto-mitral curtain and other complications. Typically, in this drawback, the tension is imposed on the sutures of the pericardial patch which reduces the life duration of the pericardial patch for the cardiac aorto-mitral curtain. These drawbacks may occur during a surgical operation, or with a possible cumulation of the problems, after a surgical operation.

A number of options is available that may reduce the tensions in the cardiac aorto-mitral curtain. An extension of the left atrial roof incision, which is necessary during valve replacement operations, into a left-right atrial trans-septal incision and the replacement of the incisions by a pericardial patch results in a transformation of the left atrial roof pericardial patch into a trans-septal atrial pericardial patch. This measure may reduce the tension and subsequent disruption of the aorto-mitral curtain. However, the technique that is required for this measure increases the complexity and duration of the operation and thus bears an additional risk for the patient.

The tension on the pericardial patch sutures of the cardiac aorto-mitral curtain may also be reduced by adding elastic biological or semi-elastic prosthetic material along the aortic valve annulus. Such a reduction of tension has been achieved in the state of the art when with an aortic valve replacement, an aortic root replacement and a replacement of the ascending aorta by an aortic prosthesis was implemented. A replacement of the aortic valve, an aortic root replacement and a replacement of the ascending aorta by an aortic prosthesis has been reported by Bentall (Bentall H., DeBono A., "A technique for complete replacement of the ascending aorta", Thorax, volume 23, pages 338-339, 1968). In a preferred embodiment, an entirely biological homograft may be used for this technique, as described by Quintana (Quintana et al., Ann. Cardiothorac. Surg., "Infective aortic valve endocarditis with root abscess formation: a mitral sparing root-Commando operation", volume 8 (6), pages 711-712, 2019, doi: 10.21037/acs.2019.06.09). By these measures, the tension on the pericardial patch sutures of the cardiac aorto-mitral curtain may be reduced.

The tension on the pericardial patch sutures may also be reduced by implanting the aortic valve prosthesis significantly above the anatomic aortic valve annulus, high in the ascending aorta. The cardiac aorto-mitral-curtain prosthesis that is used then has to support the mitral valve only and is less subjected to tension and disruption. This technique has been disclosed by Cabrol et al. (Cabrol et al., J. Thorac Cardiovasc. Surg., "Complete replacement of the ascending aorta with reimplantation of the coronary arteries: new surgical approach", volume 81, pages 309-315, 1981). For this operation, a tubular prosthesis of Dacron-material is used. This technique, however, changes the anatomy of the aortic valve, which is often undesired for the further physical wellness of the patient. Furthermore, this technique increases the complexity and duration of the surgical procedure and thus bears an additional risk for the patient.

The techniques that are listed above may partially alleviate the problem of tension in the sutures and in the pericardial patch that replaces the cardiac aorto-mitral curtain. However, they increase the complexity and duration of the cardiac operations. Furthermore, the techniques that are mentioned above aim to reduce the technical drawbacks of the existing procedure for the replacement of the cardiac aorto-mitral curtain. All procedures that have been described replace the central part of the intervalvular fibrous body only. They do not aim at performing an anatomical reconstruction of the entire cardiac aorto-mitral curtain. The intervalvular fibrous body including the right and left trigons is left untreated.

Therefore, means are searched to decrease the operative risk for patients who require a replacement of the cardiac aorto-mitral curtain during surgical operations on the mitral valve or on the aortic valve of the human heart. Furthermore, means are searched to provide a pericardial patch that reduces the risk of tensions and disruptions in the human heart during and after a surgical operation with a replacement of the intervalvular fibrous body.

The person skilled in the art has the objective to supply means for the replacement of the intervalvular fibrous body by a cardiac aorto-mitral curtain during a surgical operation on the human heart with reducing the risk of tensions and disruptions of the replaced cardiac aorto-mitral curtain and with reducing the operational risk for the patient.

The current patent application solves this objective by a pericardial patch that serves as a cardiac aorto-mitral prosthesis that replaces the intervalvular fibrous body by a cardiac aorto-mitral curtain of the human heart with a shaping that matches the anatomic shape of the intervalvular fibrous body including the left and right fibrous trigons.

By this cardiac aorto-mitral prosthesis that replaces the cardiac aorto-mitral curtain of the human heart in its anatomic shape, the risk of tensions and disruptions is largely eliminated, the complexity of the operation is reduced, the operation time is shortened, the surgical result is improved and complications are obviated. Furthermore, the positioning of the replaced or repaired aortic and mitral valves is optimized and the annular support of the implanted artificial valves improves postoperative cardiac function and increases the chances for a successful surgical repair remarkably.

During the surgical operation, the present invention can be implanted through both an aortic-left atrial roof surgical approach or through an aortic-trans-septal approach by a surgical incision of the left atrial roof that continues into the right atrial roof.

The cardiac aorto-mitral prosthesis is also easy to be produced which makes it versatile for the surgeon.

The current patent application primarily claims a cardiac aorto-mitral prosthesis for the human heart, consisting of a pericardial patch, forming a template of the aorto-mitral curtain, comprising
- two leaflets separated by a mental partition that is a central line and separates the cardiac aorto-mitral prosthesis into a first leaflet and a second leaflet for a placement of the cardiac aorto-mitral prosthesis between the aortic valve and the mitral valve during a surgical operation to the human heart, and
- one first leaflet having a first leaflet recess which serves for the inclusion of the aortic valve annulus, and the other second leaflet having a second leaflet recess which serves for the inclusion of the mitral valve annulus, and
- the cardiac aorto-mitral prosthesis having over both leaflets one larger recess for the support to the tricuspid valve annulus, and
- on the opposite side of the larger recess over both leaflets an outer edge which forms a support to the frame of the inner layer of the atrio-ventricular junction of the human heart.

Dependent on the surgical problem and the placing of the cardiac aorto-mitral prosthesis, the cardiac aorto-mitral prosthesis allows an anatomical restoration of the aorto-mitral angle between the mitral valve and the aortic valve. This measure also reduces the risk of a left ventricular outflow tract obstruction. The first leaflet and the second leaflet of the aorto-mitral prosthesis assure complete anatomic replacement of the intervalvular fibrous body including the left and the right fibrous trigons.

In an important embodiment of the invention, the mental partition that separates the cardiac aorto-mitral prosthesis into the first leaflet and the second leaflet between the aortic valve and the mitral valve of the human heart is marked by a visible line. The visible line marks the line of contact of the cardiac aorto-mitral prosthesis between the insertion of the wall of the left coronary and of the acoronary sinus of the aortic root and the insertion of the left atrial roof into the cardiac aorto-mitral curtain. By this measure, time is saved during the operation to the human heart which is often of critical importance. In an embodiment, the visible line is marked on the pericardial patch by a color that is physiologically inert. Colors that are physiologically inert are known to the person skilled in the art. The mental partition is usually centrally arranged between the first leaflet and the second leaflet. The mental partition is a central line that separates the prosthesis into two annular parts between the aortic valve and the mitral valve of the human heart.

In another embodiment, the mental partition is marked by a folding instead of a color. The folding obviates the use of a color which is useful in cases where colors are unsuitable when an immunological reaction is expected. This may apply for instance for patients that are prone to immunological reactions or for cases that a physiologically inert color for the marking is not available. A marking by a suturing is also feasible.

In still another embodiment of the invention, the cardiac aorto-mitral prosthesis has markings present on the cardiac aorto-mitral prosthesis that mark the positions of at least one commissure of the aortic valve or of the mitral valve. These markings mark the positions of the commissures in the artificial valves that are to be implanted into the human heart during the surgical operation. This helps for an alignment of the artificial valves. The commissures that may be aligned by the marking are for instance the left coronary commissure, the left acoronary commissure, the right coronary commissure or the right acoronary commissure or a combination of these commissures of the aortic valve. This commissure may also be the right commissures, the left commissures, or the center of the anterior annulus or a combination of these commissures of the mitral valve. By the markings, the surgeon may quickly and appropriately align the cardiac aorto-mitral prosthesis with the markings on the pericardial patch during the surgical operation in correspondence to the anatomic position of the commissures in the human heart. This saves additional time and improves the functional results of the surgical reconstruction.

In a further important embodiment, the pericardial patch that serves as a cardiac aorto-mitral prosthesis may be extended by another further third leaflet that is attached to the cardiac aorto-mitral prosthesis along the mental partition and serves for the replacement or enlargement of either the wall of the aortic root or the wall of the ascending aorta. The third leaflet is typically attached by a suturing. By this further third leaflet, a cardiac aorto-mitral prosthesis that comprises three leaflets is then provided.

The replacement or enlargement of either the aortic wall, the aortic root or of the ascending aorta during surgical operations on the human heart is known in the state of the art. It is usually accomplished by a single pericardial patch without further attached pericardial patches and is also known as the "Manouguian-type"-reconstruction or enlargement (S. Manouguian, P. G. Kirchhoff, Ann. Thorac Surg. "Patch enlargement of the aortic and the mitral valve rings with aortic-mitral double-valve replacement", volume 30 (4), pages 396-399, 1980). The cardiac aorto-mitral curtain of the current invention may be easily appended with the "Manouguian-type"-reconstruction or enlargement by attaching the third leaflet to the cardiac aorto-mitral prosthesis along the mental partition.

The use of the further third leaflet is indicated when the damage to the heart is extending also to the wall of the aortic root or the wall of the ascending aorta or when an aortic root enlargement is required. The further third leaflet consists of pericardial patch. A separate repair of the wall of the aorta by a pericardial patch is then obviated so that time during the operation is saved and the risk of a misplacement in relation to the cardiac aorto-mitral curtain and the wall of the aortic root or the ascending aorta is lowered. When the Bentall-procedure as described above is used to replace the aortic valve, root and wall, then the third leaflet may be also used for the reconstruction, respectively the closure of the left atrial roof incision.

In a further important embodiment, the pericardial patch that serves as a cardiac aorto-mitral prosthesis may be extended by another further fourth leaflet that is also attached to the cardiac aorto-mitral prosthesis along the mental partition. With the fourth leaflet, the third leaflet is used to enlarge the aortic annulus and root and the fourth leaflet is used to replace or close the left atrial roof at the location of the atrial incision. The fourth leaflet is typically attached by a suturing. By this further fourth leaflet, a cardiac aorto-mitral prosthesis that comprises four leaflets is then provided. The fourth leaflet is typically used for the closure or the enlargement of the left atrial roof at the location of the surgical incision. Like the third leaflet, the fourth leaflet consists of a pericardial patch. The use of a the cardiac aorto-mitral prosthesis that comprises the fourth leaflet is indicated when a surgical incision into the left atrial roof is implemented to expose and perform surgery on the mitral valve or on the aortic valve, and when a replacement of the cardiac aorto-mitral curtain is necessary. This is frequently the case with damage to the heart that is extending deeply into the intervalvular fibrous body and into the mitral valve annulus.

By the fourth leaflet, a separate repair of the roof of the left atrium by a separate pericardial patch is obviated so that time during the operation is saved and the risk of a misplacement of the pericardial patch for the left atrial roof in relation to the cardiac aorto-mitral curtain is lowered. Inserting a cardiac aorto-mitral prosthesis with four leaflets replaces the anatomic aorto-mitral curtain, replaces the wall of the ascending aorta or the wall of the aortic root and closes or enlarges the left atrial roof at the location of the surgical incision. The 360°-mobility of the four leaflets in relation to each other and the mobility of the anterior mitral annulus in relation to the left-coronary or a-coronary aortic valve annulus accomplishes the re-establishment of the natural anatomical and individual aorto-mitral angle. This further reduces the tension on the surgical sutures and optimizes the left ventricular function by independently maintaining the aortic and mitral annular excursions and preventing left ventricular outflow obstruction. Furthermore, the 360°-mobility of the four leaflets in relation to each other and the mobility of the anterior mitral valve annulus in relation to the left-coronary or a-coronary aortic valve annulus stabilizes the excursion of the aortic and mitral valve annulus during the cardiac cycle.

The further fourth leaflet that is attached to the cardiac aorto-mitral prosthesis along the mental partition for a closure or an enlargement of the left atrial roof may also be used instead of the third leaflet for the covering of the wall of the ascending aorta so that a cardiac aorto-mitral prosthesis that comprises three leaflets is provided. The use of this pericardial patch with only the fourth leaflet as an additional leaflet to the first and second leaflet of the cardiac aorto-mitral prosthesis is especially indicated when the procedure that was described by Bentall et al. with an aortic valve prosthesis and a replacement of the entire aortic root with the ascending aorta and a replacement of the cardiac aorto-mitral curtain with the cardiac aorto-mitral prosthesis is implemented. In these cases, the replacement of the left atrial roof is often necessary. The procedure, as described by Bentall et al., with the cardiac aorto-mitral prosthesis may also be applied in the embodiment as described by Quintana, with an aortic homograft.

For the embodiment of the cardiac aorto-mitral curtain with a third leaflet and a further fourth leaflet, the different forms of the third leaflet and the further fourth leaflet allow distinction between the aortic part and the left atrial part of the cardiac aorto-mitral curtain, thus allowing correct positioning and implantation of the aorto-mitral prosthesis during the surgical operation. By the different shapes of the triangular embodiment with three leaflets and the quadrangular embodiment with four leaflets, the different shapes allow distinction between the left atrial part and the aortic part of the cardiac aorto-mitral prosthesis, thus allowing correct positioning and rapid implantation of the aorto-mitral prosthesis during the surgical operation.

In a further embodiment of the invention, the cardiac aorto-mitral prosthesis has a first leaflet extension that extends the ending of the right aortic annular part that includes the aortic valve and supports the tricuspid valve annulus. The use of the first leaflet extension is indicated when the damage to the heart is extending over the right fibrous trigone to the entire intervalvular fibrous body and the right annular part of the aortic valve and the annulus of the tricuspid valve. This kind of damage is frequent when an inflammation of the aortic annulus extends through the central fibrous body into the annulus of the aortic valve and into the annulus of the tricuspid valve.

By the use of a pericardial patch that is used as a cardiac aorto-mitral prosthesis with a first leaflet extension, the entire central fibrous body with the right annular part of the aortic valve and the annulus of the tricuspid valve is replaced, and the implantation of a separate pericardial patch that replaces the right annulus of the aortic valve and supports the tricuspid valve annulus can be avoided. This prevents misplacement of a separate pericardial patch for the replacement of the right aortic annular part that includes the aortic valve and supports the tricuspid valve annulus. Time is saved during the surgical operation to the human heart and a complete replacement of the entire central fibrous body of the heart is achieved. Furthermore, the anatomic replacement of the aorto-mitral angle is achieved, physiological excursion is allowed and the function of the aortic and mitral valve annuli is restored which reduces the risk of a left ventricular outflow obstruction. Furthermore, the cardiac aorto-mitral prosthesis with a first leaflet extension facilitates correct positioning of the aortic valve in relation to the mitral valve and, in addition, facilitates the insertion of the cardiac aorto-mitral prosthesis, which reduces the risk of suture disruption and bleeding after surgery.

In this embodiment with a first leaflet extension, a third leaflet or a fourth leaflet or a third leaflet and a fourth leaflet may be attached to the cardiac aorto-mitral prosthesis. By attaching a third leaflet or a fourth leaflet to the cardiac aorto-mitral prosthesis, a mechanical stabilization of both the annulus of the aortic valve and the annulus of the tricuspid valve is achieved during the entire cardiac cycle.

The first leaflet extension may be marked by a folding or by a visible line in separation from the ending of the first leaflet that includes the aortic valve and supports the tricuspid valve annulus. By the marking or the folding, the extension may properly replace the part of the fibrous central body situated between the aortic valve annulus and the tricuspid valve annulus and also support the tricuspid valve annulus during the operation. This further minimizes the risk of distortion and rupture. The first leaflet extension may be cut and formed quickly into the required shape in order to fit and replace the removed part of the tissue in the case that during the operation, the damage to the aortic valve annulus turns out not to have proceeded or to have proceeded to a lesser than expected extent.

In a further embodiment of the invention, the cardiac aorto-mitral prosthesis has a recess extension over the recess ending of the second leaflet in separation from the unextended cardiac aorto-mitral prosthesis of the pericardial patch of the cardiac aorto-mitral prosthesis over the anterior cusp of the mitral valve annulus. By this recess extension, a proceeding damage into the anterior mitral valve annulus may be replaced, with preservation of the natural mitral valve coaptation, and preservation of the entire posterior cusp of the mitral valve. This proceeding damage may be for instance an inflammation, such as an endocarditis of the cardiac aorto-mitral curtain that involves the anterior cusp of the mitral valve. By this measure, a patch with a recess extension is kept in stock which may be altered by more or less cutting to the pericardial patch without recess extension in the case that after access to the human heart and surgical exposure of the mitral valve, the damage to the mitral valve annulus turns out to be more or less extensive and would require mitral valve replacement. This embodiment may also be indicated in clinical cases of a massive left atrial enlargement, where the fourth leaflet of the prosthesis is not required and a reduction of the left atrial roof can be implemented instead.

The replacement of the cardiac aorto-mitral curtain associated with aortic valve reconstruction has been described in the state of the art by Quintana et al. (Quintana et al., Ann. Cardiothorac. Surg., "Infective aortic valve endocarditis with root abscess formation: a mitral sparing root-Commando operation", volume 8 (6), pages 711-712, 2019, doi: 10.21037/acs.2019.06.09). One elliptical pericardial patch was used to reconstruct the left atrial roof and the non-coronary sinus of the aortic root and wall. This method is also called the "hemi-Commando"-operation. By the extension of the replacement to the aortic valve annulus with a pericardial patch, a so-called "hemi-conduit" is formed. This operation was also suggested for a mitral valve replacement. For the aortic valve annulus replacement, the same method was applied as in the original method for the replacement of the cardiac aorto-mitral curtain, as described by T. E. David et al. Quintana et al. however, did not reconstruct the cardiac aorto-mitral curtain and their method did not extend to the intervalvular fibrous body including the left and right fibrous trigons, as in the currently described invention. A mitral valve annulus replacement was not described.

In an embodiment of the cardiac aorto-mitral prosthesis with the recess extension, the recess extension is marked by a visible line over the recess ending in separation from the unextended cardiac aorto-mitral prosthesis. The extension may be cut away by the surgeon during the operation so that a regular cardiac aorto-mitral prosthesis without recess extension is formed. By the marking, the extension may be cut away quickly into the required shape in order to replace the removed part of the tissue in the case that during the operation, the damage to the mitral valve annulus turns out not to have proceeded or to have proceeded to a lesser than expected extent. As with the marking of the first leaflet extension, the markings may be applied with a physiologically inert color or a folding.

In a further embodiment of the invention, an artificial mitral valve prosthesis is attached to the cardiac aorto-mitral prosthesis. By this measure, a cardiac aorto-mitral prosthesis equipped with an attached artificial mitral valve is provided. The attachment is typically carried out by a suturing. By attaching the artificial mitral valve to the cardiac aorto-mitral prosthesis, the artificial mitral valve may be directly inserted into the human heart for the replacement of the mitral valve in the case that the natural mitral valve is damaged and must be replaced. Attaching the mitral valve annulus valve to the cardiac aorto-mitral prosthesis saves time during the surgical operation and makes the surgical operation simpler and more precise for the replacement of the cardiac aorto-mitral curtain and the mitral valve. A further attachment of an artificial aortic valve for the replacement of the innate aortic valve, although less frequently carried out, also falls within the range of the current invention.

The first and the second leaflet may be supplied with markings for the left-noncoronary and right-noncoronary commissures and a marking for the center of the anterior mitral cusp of the mitral valve prosthesis. This supports an alignment of mitral valve prosthesis during the surgical operation so that time may be saved and the precision of the surgical operation is improved.

The double replacement of the mitral valve and of the aortic valve with the replacement of the aorto-mitral curtain with the markings on the first and the second leaflet is very advantageous. The correct orientation of the artificial mitral valve prosthesis is often difficult after a resection of the cardiac aorto-mitral curtain, because the positioning of the mitral valve prosthesis depends on the orientation of the aortic valve. The orientation of the aortic valve cannot be changed, because it further depends on the positioning of the origins of the coronary arteries. The implantation of the mitral valve prosthesis is usually carried out before the implantation of the aortic valve prosthesis with the effect that the mitral valve prosthesis for implantation has no point of reference for the positioning of the mitral valve commissures. By a marking of the left-noncoronary and right-noncoronary commissures and a marking of the center of the mitral anterior cusp of the mitral valve prosthesis on the first and second leaflet, perfect orientation and flawless anatomical positioning of the mitral valve prosthesis and the following aortic valve prosthesis is possible. By the perfect orientation and the flawless anatomical positioning of the mitral valve prosthesis and aortic valve prosthesis, the optimal function of left ventricular outflow tract is restored.

The cardiac aorto-mitral prosthesis is typically made of a bovine pericardial patch, such as the Model 4700 (10 cm x 15 cm) manufactured by Edwards Lifesciences, Irvine, California, USA. However, any other suitable pericardial patch for a surgical operation may be used for the cardiac aorto-mitral prosthesis of the current invention. This may be any other bovine pericardial patch produced for surgical implantation, or a Dacron^{®}-fabric. Bovine pericardial patch is a standard material for vascular biological patches and allows an easy production of the pericardial patch necessary for the cardiac aorto-mitral prosthesis. Dacron^{®} is a polyester graft that is often used in surgery. Both materials bovine pericardium and Dacron^{®} are well-known to the person skilled in the art and are readily available on the market. A Teflon-fabric is also suitable for the pericardial patch of the cardiac aorto-mitral prosthesis.

The current patent application also claims a process for the production of the cardiac aorto-mitral prosthesis that has been described above, which is characterized in that
- a pericardial patch is taken and cut into two leaflets separated by a mental partition of the cardiac aorto-mitral prosthesis, and
- into one first leaflet a first leaflet recess is cut which serves for the inclusion of the aortic valve annulus, and
- into the other second leaflet a second leaflet recess is cut for inclusion of the mitral valve annulus, and
- into the prosthesis one larger recess is cut over both leaflets for the support of the tricuspid annulus, and
- into the opposite side of the larger recess an outer edge is cut which forms a support to the inner layer of the atrio-ventricular junction of the human heart.

For the process of the production of the cardiac aorto-mitral prosthesis, a pericardial patch of an arbitrary size is cut to the shape of the cardiac aorto-mitral prosthesis. The process for the production of the cardiac aorto-mitral curtain may be carried out for instance by medical scissors or by a scalpel. The process for the production of the cardiac aorto-mitral prosthesis may be carried out before or during the surgical operation. It may also be carried out in an industrial scale.

By the process for the production of the cardiac aorto-mitral prosthesis, the aorto-mitral prosthesis with two leaflets may be easily narrowed and shortened during the surgical operation of the human heart so that it becomes perfectly adjusted to the size and form of the portion of the central fibrous body that is excised during the surgical procedure of the heart and requires replacement. This anatomical reconstruction lowers the risk of bleeding and disruption.

The size of the cardiac aorto-mitral prosthesis is determined by the maximal size of an aortic valve prosthesis and the maximal size mitral valve prosthesis. An aortic valve prosthesis has a maximal diameter of 29 mm and a mitral valve prosthesis has a maximal diameter of 33 mm. The size of the aorto-mitral prosthesis can be adjusted by reducing the pericardial patch during the surgical operation to the dimensions of the missing segment of the aorto-mitral curtain. The adjustment of size may be carried out before or during the surgical operation, although an adjustment before the surgical operation is preferred to ensure a sufficient precision. The prosthesis may for instance be produced in adaption to different valve sizes before the operation. As an example, one sample is then compatible for an aortic valve size of 21 mm and a mitral valve size of 25 mm, and another sample is compatible for an aortic valve size of 23 mm and a mitral valve size of 27 mm, another sample is compatible for an aortic valve size of 25 mm and a mitral valve size of 29 mm, and a last sample is compatible to an aortic valve size of 27 mm and a mitral valve size of 31 mm.

The cardiac aorto-mitral prosthesis that has been prepared may be stored like a regular pericardial patch. During the surgical operation, the aorto-mitral prosthesis can be easily narrowed by scissors to fit the size and form of the excised part of the intervalvular fibrous body of the human heart.

In an embodiment of the invention, a further third leaflet is attached to the cardiac aorto-mitral prosthesis for a covering of the wall of the aorta. By attaching a further third leaflet, a cardiac aorto-mitral prosthesis is provided that may replace the wall of the aortic root and of the ascending aorta corresponding to the left coronary sinus and the acoronary sinus of the aortic root. By attaching the third leaflet to the cardiac aorto-mitral prosthesis, a cardiac aorto-mitral prosthesis with three leaflets is provided. The further third leaflet is preferably attached along the mental partition. This pericardial patch with three leaflets is then a triangular cardiac aorto-mitral prosthesis. The attachment is preferably carried out by suturing. The third leaflet may be adjusted into a shape that fits to the portion of the aortic root and wall that is missing and has to be replaced.

In a further embodiment of the invention, a further fourth leaflet as a further leaflet for a covering of the left atrial roof is attached to the cardiac aorto-mitral prosthesis. By attaching a further fourth leaflet, a cardiac aorto-mitral prosthesis is provided that may cover or enlarge the left atrial roof at the location of the atrial incision. By attaching the fourth leaflet to the cardiac aorto-mitral prosthesis, a cardiac aorto-mitral prosthesis with four leaflets is provided. The further fourth leaflet is preferably attached along the mental partition. This pericardial patch with three leaflets is then a quadrangular cardiac aorto-mitral prosthesis. The attachment is preferably carried out by suturing. The fourth leaflet may also be attached to the cardiac aorto-mitral prosthesis as another leaflet instead of the third leaflet. In this latter case, also a pericardial patch with three leaflets with the fourth leaflet is provided. The fourth leaflet may be adjusted into a shape that fits to the shape and direction of the left atrial surgical incision, and for a surgical reconstruction of the left atrial roof.

The resizing of the third and the fourth leaflet may be accomplished for instance by scissors or a scalpel.

The cardiac aorto-mitral prosthesis may also be produced by leaving a recess extension in the second leaflet recess of the pericardial patch over the mitral valve annulus in the cutting of the second leaflet recess for the mitral valve annulus. The recess extension in the pericardial patch of the cardiac aorto-mitral prosthesis over the mitral valve annulus may replace the basis of the annulus that comprises the mitral valve or the basis of its anterior cusp during the surgical operation. By this recess extension, a proceeding damage into the mitral valve annulus, possibly involving the anterior cusp, may be replaced. This proceeding damage may be for instance an inflammation. The recess extension may be cut away by the surgeon during the surgical operation when the damage in the mitral valve annulus requires mitral valve replacement.

The cardiac aorto-mitral prosthesis may also be produced by integrating a mitral valve prosthesis into the cardiac aorto-mitral prosthesis and thus providing a cardiac aorto-mitral curtain prosthesis with an attached mitral valve prosthesis. The attached mitral valve prosthesis is preferably artificial as a mechanical valve or as a biological valve. The cardiac aorto-mitral curtain prosthesis with an attached mitral valve prosthesis can be directly inserted into the human heart for the replacement of the mitral valve in the case that the innate mitral valve is damaged and must be replaced together with the replacement of the cardiac aorto-mitral curtain. The cardiac aorto-mitral curtain prosthesis with an attached aortic valve prosthesis can be directly inserted into the human heart for the replacement of the aortic valve in the case that the innate aortic valve is damaged and must be replaced together with the replacement of the cardiac aorto-mitral curtain.

The cardiac aorto-mitral prosthesis of the current invention may preferably be used for a surgical operation to the human heart. It is, however, conceivable that the cardiac aorto-mitral prosthesis is used also for animal hearts with a comparably anatomy.

The invention has the advantage that a cardiac aorto-mitral prosthesis is provided with a pericardial patch that decreases the operational risk for the patient and that reduces the risk of tensions and disruptions for the aorto-mitral curtain and the intervalvular fibrous body of the heart. Furthermore, the pericardial patch can be placed more easily and properly during the surgical operation so that the operational risks during the surgical operation is reduced for the surgeon. Time may be saved by the cardiac aorto-mitral prosthesis that has been described which further reduces the risk during the surgical operation. Further surgical operations become unnecessary and are obviated which is of advantage for the health and well-being of the patient. Finally, the cardiac aorto-mitral prosthesis is easy to be produced which makes it versatile for the surgeon.

The invention is further described by fifty-six drawings. The drawings only show examples and embodiments of the invention and do not limit the scope of the invention. The cardiac aorto-mitral prosthesis is a pericardial patch that replaces the intervalvular fibrous body of the heart.

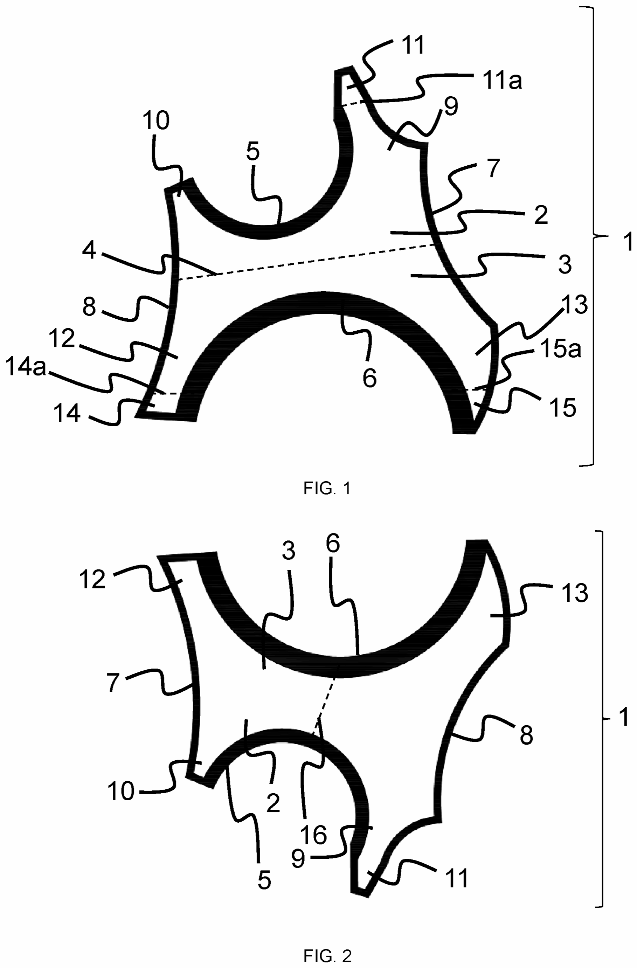

The drawing FIG. 1 shows the cardiac aorto-mitral prosthesis according to the claimed invention with the first and the second leaflet separated by the mental partition in a view from above.

The drawing FIG. 2 shows the cardiac aorto-mitral prosthesis according to the claimed invention with two leaflets separated by a lengthwise mental partition turned by 180° in a view from above.

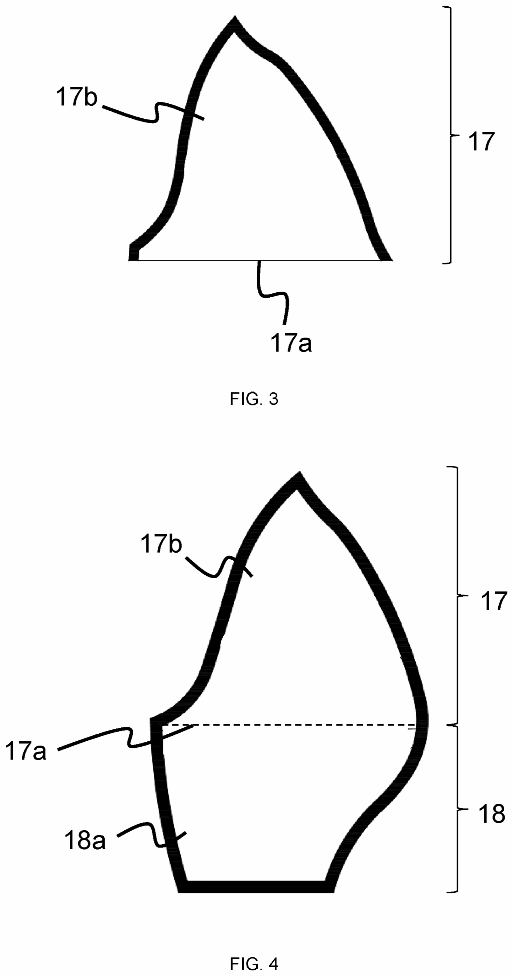

The drawing FIG. 3 shows a third leaflet as state of the art for the replacement of the aortic wall and the aortic root in a view from above.

The drawing FIG. 4 shows the combined third leaflet for the replacement of the aorta wall and of the aortic root and a fourth leaflet as state of the art for the replacement of the left atrial roof in a view from above.

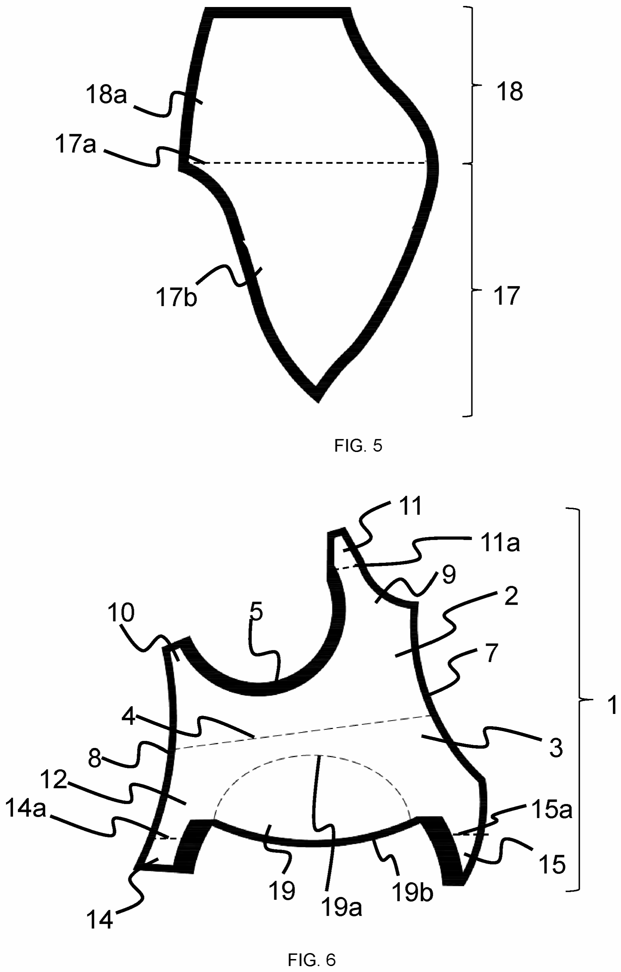

The drawing FIG. 5 shows the combined third leaflet and the fourth leaflet turned by 180° in a view from above as state of the art.

The drawing FIG. 6 shows the cardiac aorto-mitral prosthesis according to the claimed invention with a recess extension in the second leaflet recess that may be used for the replacement of the mitral valve annulus.

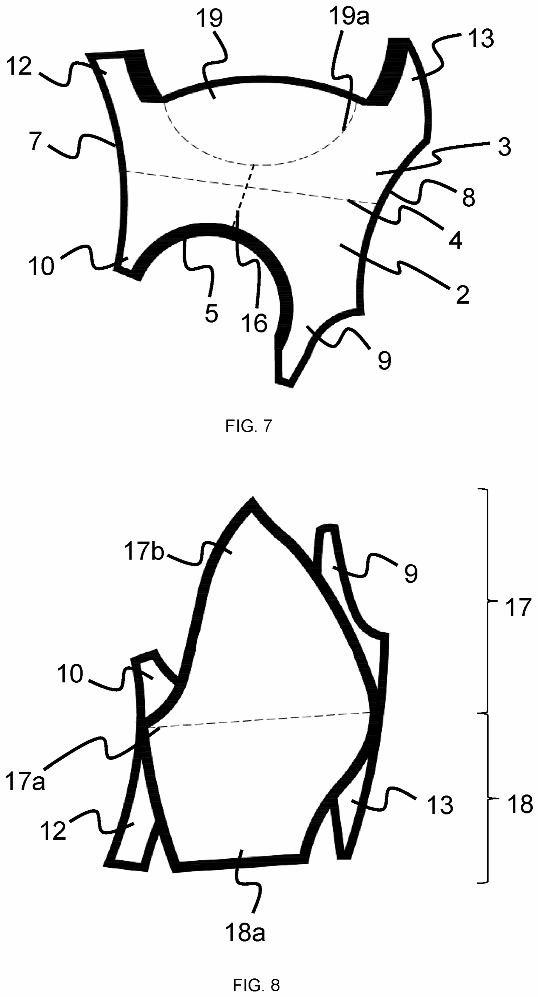

The drawing FIG. 7 shows the cardiac aorto-mitral prosthesis according to the claimed invention with two leaflets with a recess extension in the second leaflet and a lengthwise mental partition turned by 180° in a view from below.

The drawing FIG. 8 shows the cardiac aorto-mitral prosthesis according to the claimed invention in the embodiment with four leaflets in a view from above.

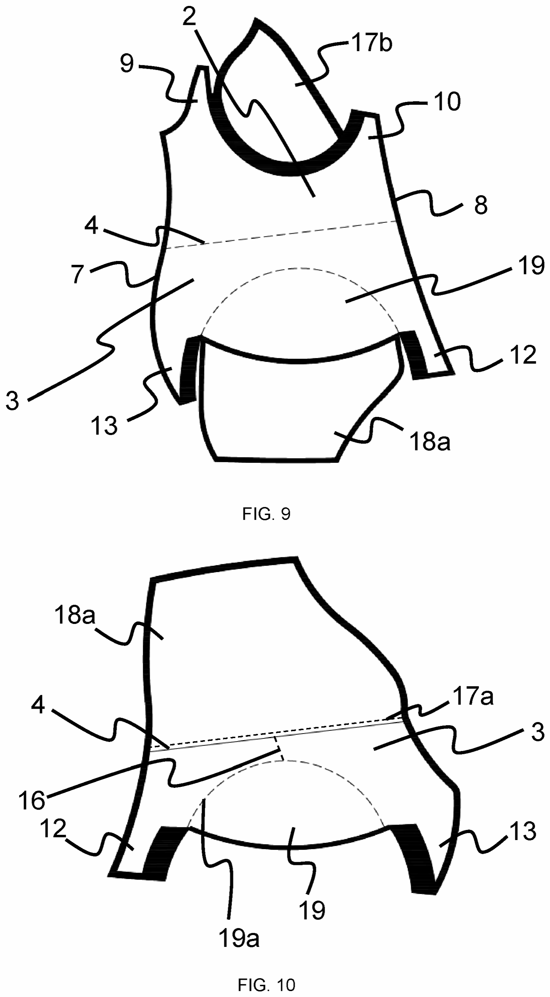

The drawing FIG. 9 shows the cardiac aorto-mitral prosthesis according to the claimed invention in the embodiment with four leaflets in a view from below.

The drawing FIG. 10 shows the cardiac aorto-mitral prosthesis according to the claimed invention with the second leaflet having a recess extension for the reconstruction of the mitral valve and a fourth leaflet for replacing the left atrial roof in a view from above.

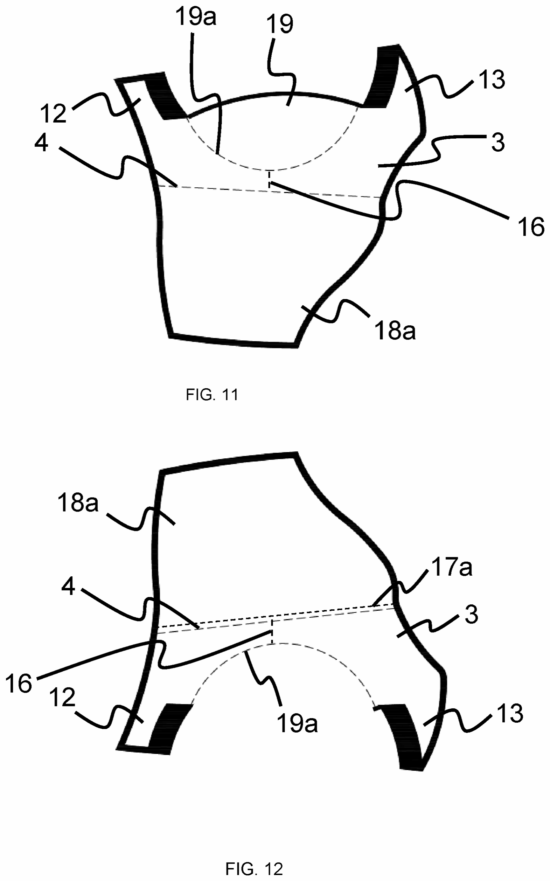

The drawing FIG. 11 shows the cardiac aorto-mitral prosthesis according to the claimed invention with the second leaflet having a recess extension for the reconstruction of the mitral valve and the fourth leaflet turned by 180° in a view from above.

The drawing FIG. 12 shows the cardiac aorto-mitral prosthesis according to the claimed invention with the second leaflet without recess extension and the fourth leaflet for the replacement of the left atrial roof and a lengthwise mental partition in a view from above.

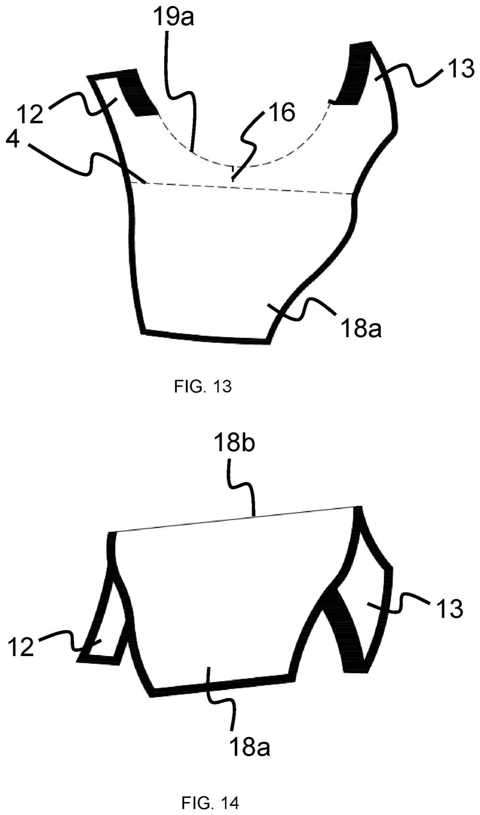

The drawing FIG. 13 shows the cardiac aorto-mitral prosthesis according to the claimed invention with the second leaflet without recess extension and the fourth leaflet for the replacement of the left atrial roof and a lengthwise mental partition turned by 180° in a view from above.

The drawing FIG. 14 shows the cardiac aorto-mitral prosthesis according to the claimed invention with the second leaflet in the background and the fourth leaflet in the foreground in a view from above.

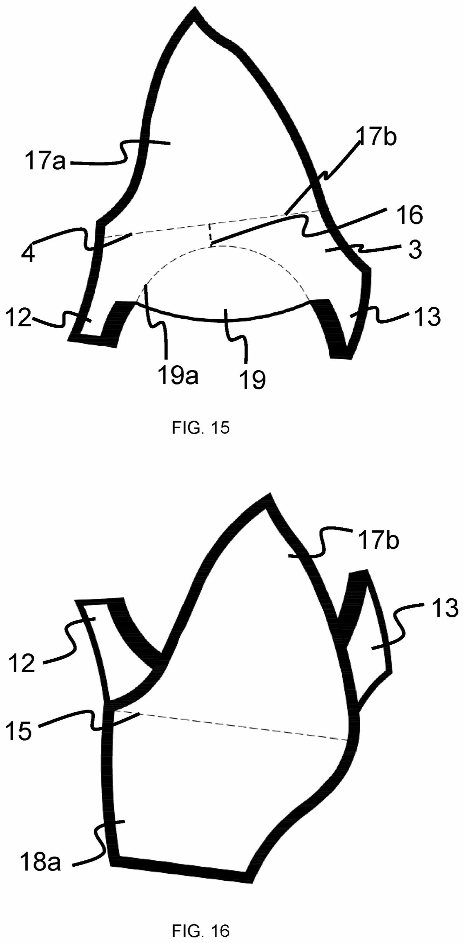

The drawing FIG. 15 shows the cardiac aorto-mitral prosthesis according to the claimed invention having a recess extension in the foreground with an attached third leaflet in a view from above.

The drawing FIG. 16 shows the cardiac aorto-mitral prosthesis according to the claimed invention with the second leaflet in the background and with the third leaflet and the fourth leaflet in the foreground in a view from above.

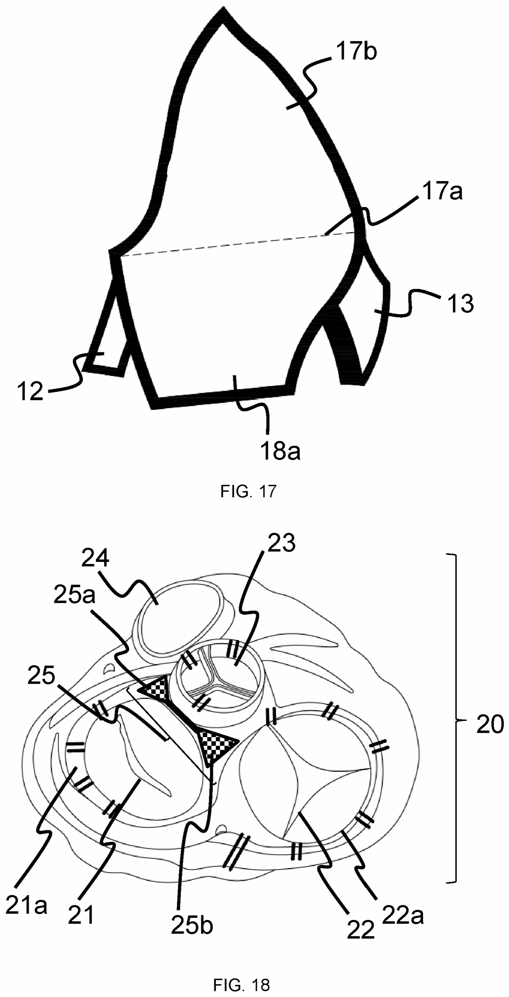

The drawing FIG. 17 shows the cardiac aorto-mitral prosthesis according to the claimed invention with the second leaflet in the background and with the third leaflet and the fourth leaflet in the foreground in a view from above.

The drawing FIG. 18 shows a drawing of the human heart transected according to the state of the art just above the atrioventricular valves as it looks after the removal of the left atrium and right atrium with its anatomy for a demonstration of the localization of the intervalvular fibrous body of the heart with the anatomic position of the left fibrous trigon and of the right fibrous trigon and the anatomic relationship to the aortic valve, mitral valve and tricuspid valve.

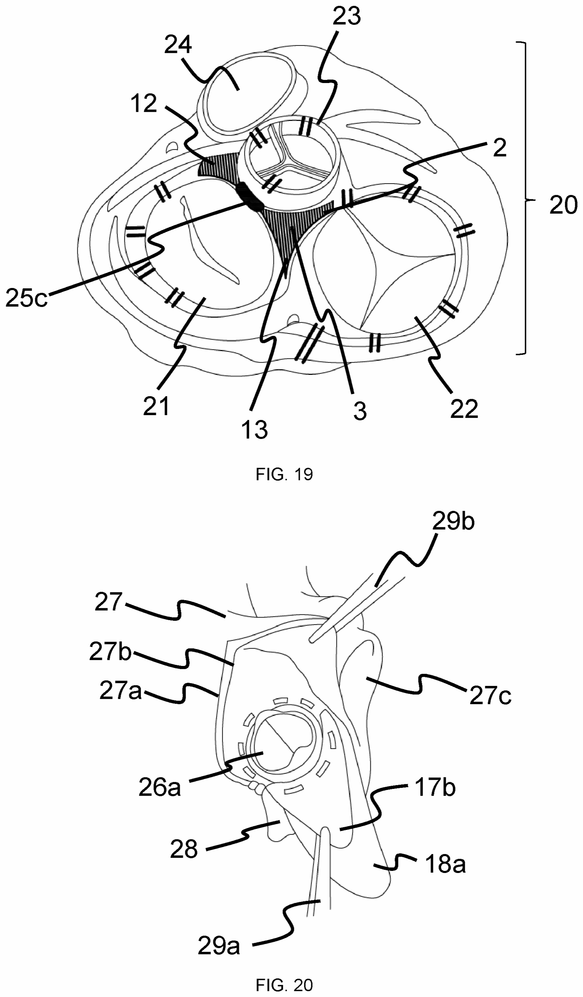

The drawing FIG. 19 shows a drawing of the human heart transected just above the atrioventricular valves as it looks after the removal of the left atrium and right atrium, with the cardiac aorto-mitral prosthesis according to the claimed invention that has been inserted for a replacement of the intervalvular fibrous body.

The drawing FIG. 20 shows the cardiac aorto-mitral prosthesis according to the claimed invention with four leaflets and an attached artificial mechanical aortic valve prosthesis during a surgical operation in a view from above.

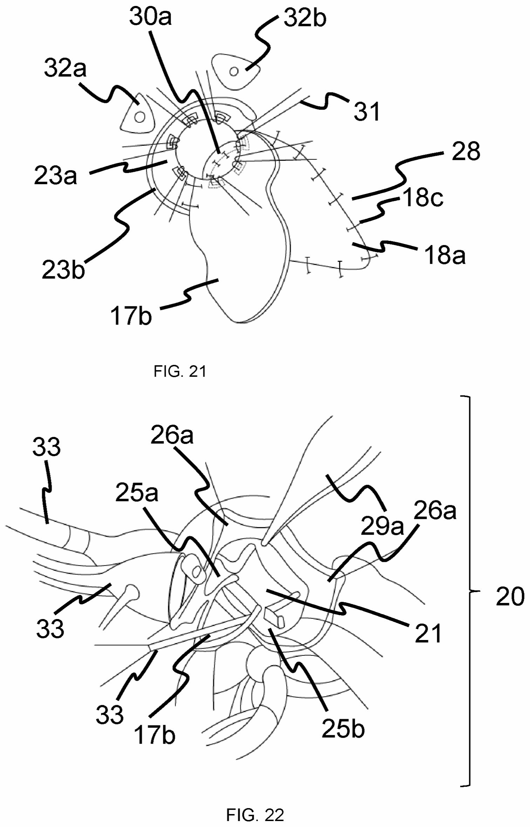

The drawing FIG. 21 shows the cardiac aorto-mitral prosthesis according to the claimed invention with four leaflets and an attached artificial mechanical mitral valve prosthesis during a surgical operation in a view from above.

The drawing FIG. 22 shows the human heart during an aortic valve replacement with the third leaflet of the cardiac aorto-mitral prosthesis according to the claimed invention for the enlargement of aortic wall during a surgical operation, when staying on the right side of the patient.

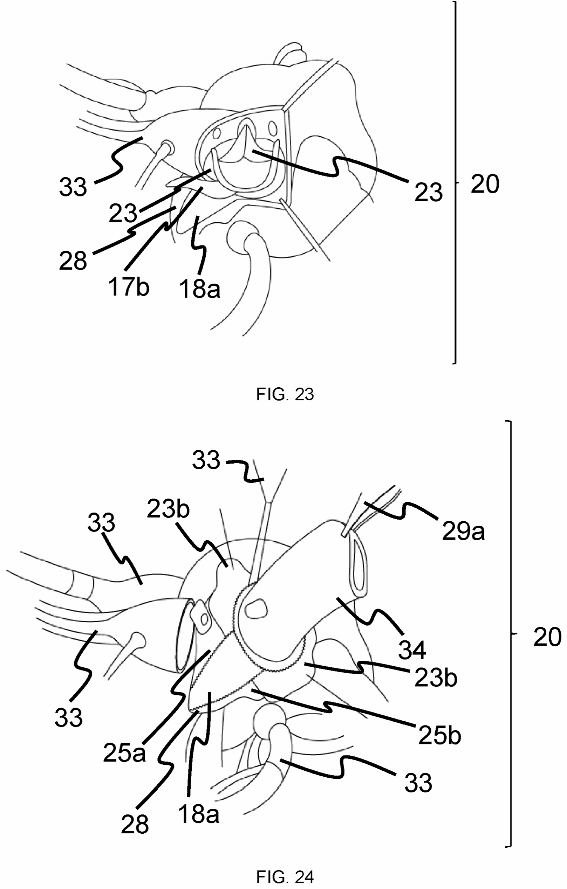

The drawing FIG. 23 shows the human heart with the replaced aortic valve by an artificial biological aortic valve prosthesis with a replaced cardiac aorto-mitral curtain by the means of the cardiac aorto-mitral prosthesis according to the claimed invention during a surgical operation, when staying on the right side of the patient.

The drawing FIG. 24 shows the human heart with the replaced aortic valve by an aortic root homograft with the cardiac aorto-mitral prosthesis according to the claimed invention and the third leaflet that covers the aortic wall and the fourth leaflet that replaces the left atrial roof with the innate anatomical left trigone and the innate anatomical right trigone in a view from above.

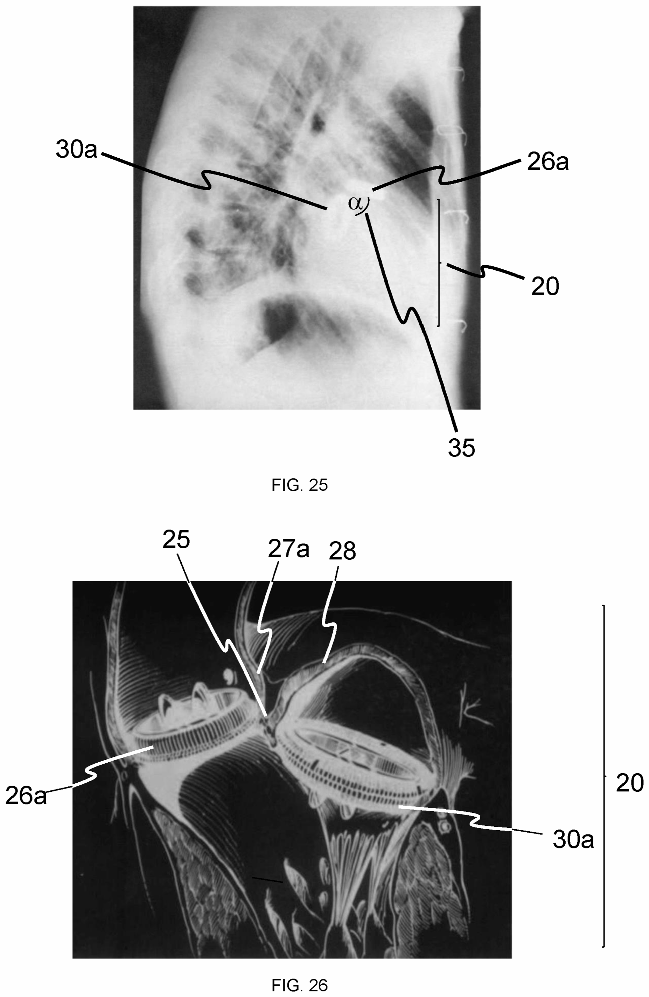

The drawing FIG. 25 shows an X-ray photography of the human heart with a visible artificial biological mitral valve and a visible artificial biological aortic valve according to the state of the art with a reestablished aorto-mitral angle α in a side view.

The drawing FIG. 26 shows an X-ray photography of the human heart with an artificial mechanical mitral valve and an artificial mechanical aortic valve according to the state of the art and the aorto-mitral curtain with a reestablished aorto-mitral angle between the valves in a side view.

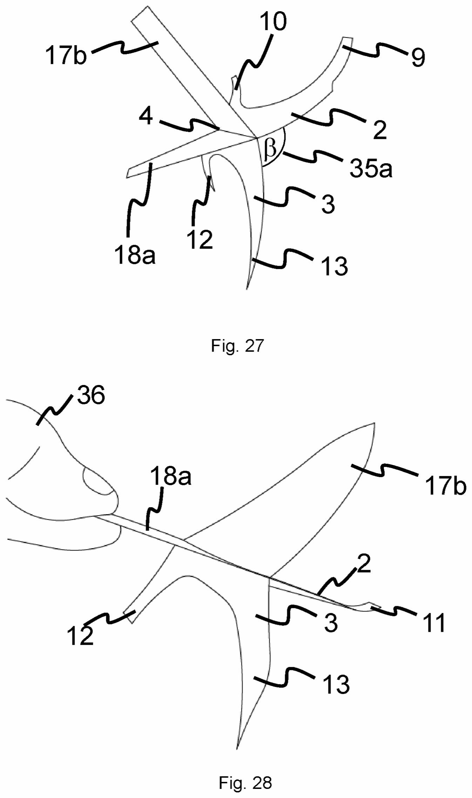

The drawing FIG. 27 shows the cardiac aorto-mitral prosthesis according to the claimed invention with an attached third leaflet and an attached fourth leaflet for implantation in a side view.

The drawing FIG. 28 shows the cardiac aorto-mitral prosthesis according to the claimed invention with an attached third leaflet and an attached fourth leaflet of the cardiac aorto-mitral prosthesis with four leaflets being kept between fingers in a side view.

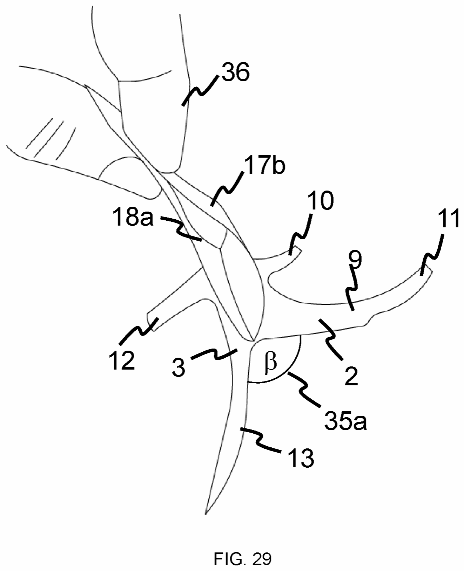

The drawing FIG. 29 shows the cardiac aorto-mitral prosthesis according to the claimed invention with the attached third leaflet and the attached fourth leaflet of the cardiac aorto-mitral prosthesis with four leaflets being kept between fingers and the stretching angle β in a side view.

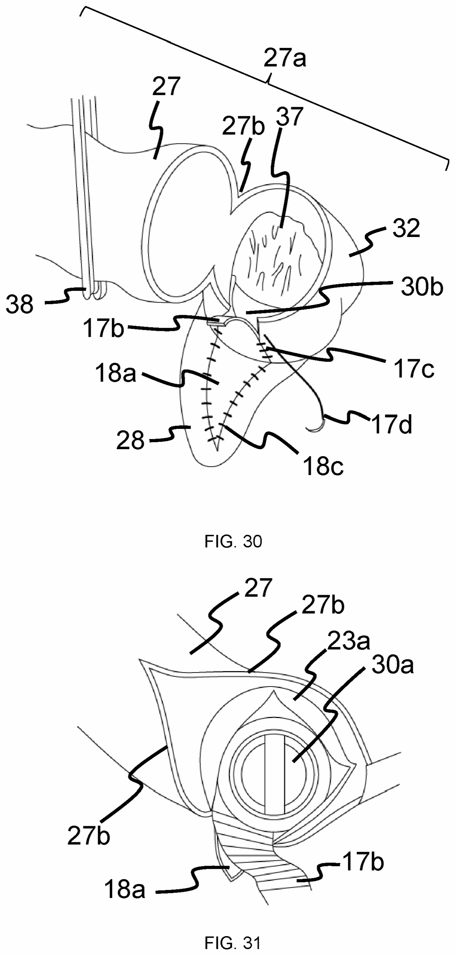

The drawing FIG. 30 shows the incision into the aorta and the aortic root of the human heart with an implanted cardiac aorto-mitral prosthesis according to the claimed invention with four leaflets and a biological mitral valve prosthesis, as seen during the surgical operation in a view from above.

The drawing FIG. 31 shows the aorta of the human heart during replacement of the wall of the aorta by a third leaflet according to the state of the art in a side view when staying on the right side of the patient.

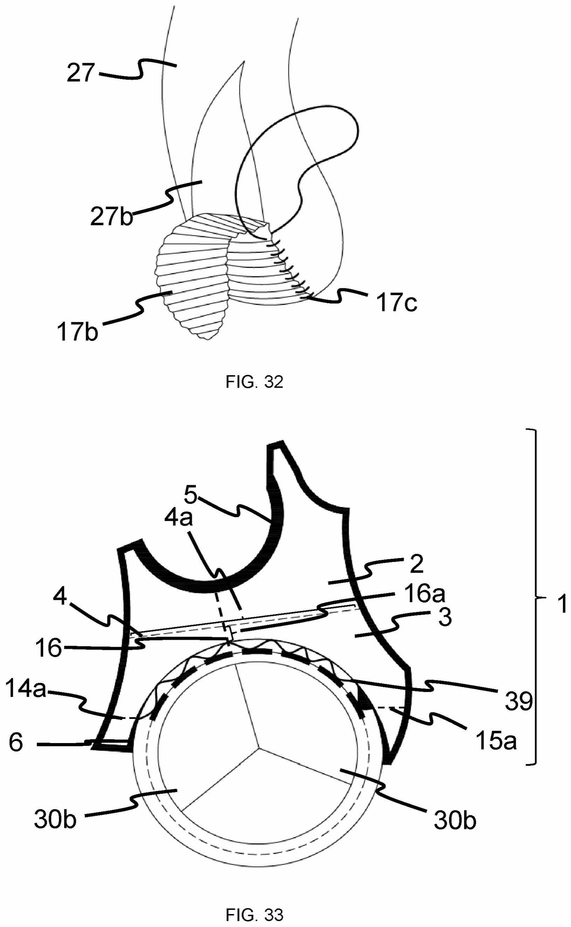

The drawing FIG. 32 shows the aorta of the human heart during replacement of the aortic wall and aortic root with a Dacron^{®}-patch according to the state of the art having the shape of the third leaflet of the cardiac aorto-mitral prosthesis in a closer view when staying on the right side of the patient.

The drawing FIG. 33 shows the cardiac aorto-mitral prosthesis according to the claimed invention with an attached artificial biological mitral valve in a view from above.

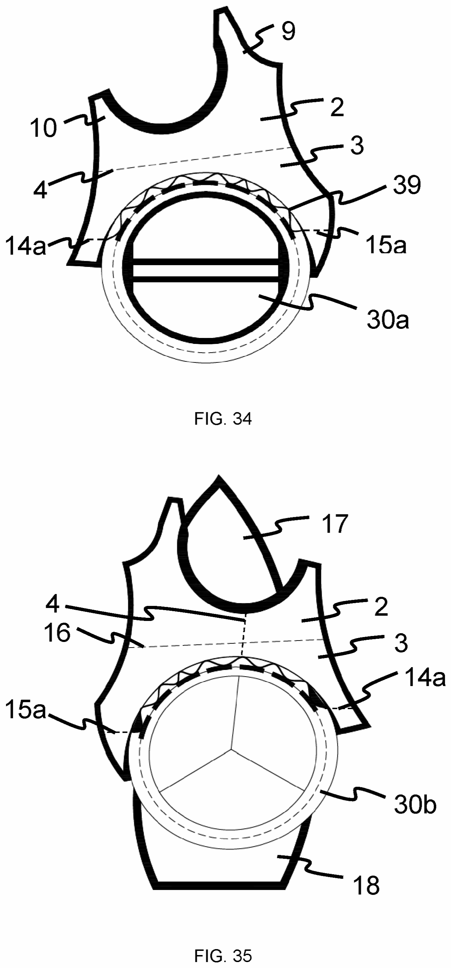

The drawing FIG. 34 shows the cardiac aorto-mitral prosthesis according to the claimed invention with an attached artificial mechanical mitral valve in a view from above.

The drawing FIG. 35 shows the cardiac aorto-mitral prosthesis according to the claimed invention with four leaflets and an attached artificial mechanical mitral valve prosthesis in a view from below.

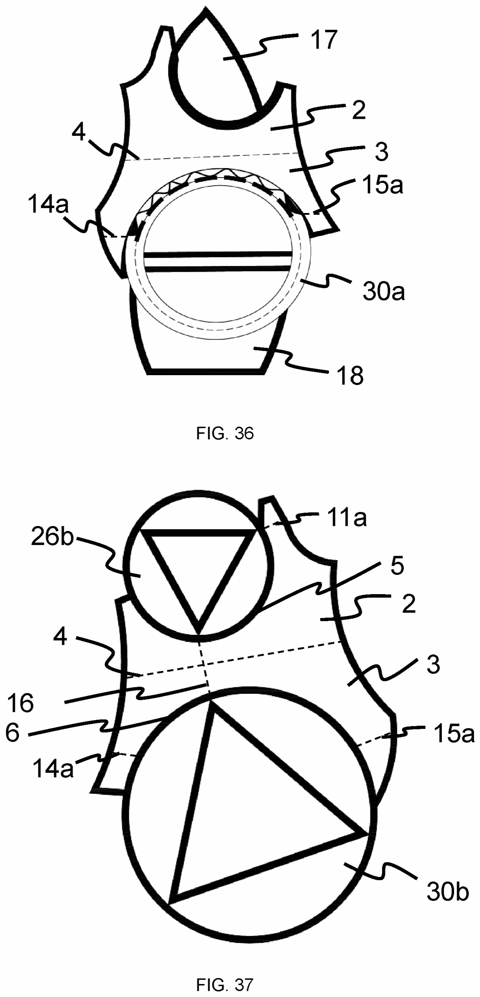

The drawing FIG. 36 shows the cardiac aorto-mitral prosthesis according to the claimed invention with four leaflets and an attached artificial biological mitral valve prosthesis in a view from below.

The drawing FIG. 37 shows the cardiac aorto-mitral prosthesis according to the claimed invention with the correct positioning of an artificial biological mitral valve and an attached artificial biological aortic valve in a view from above.

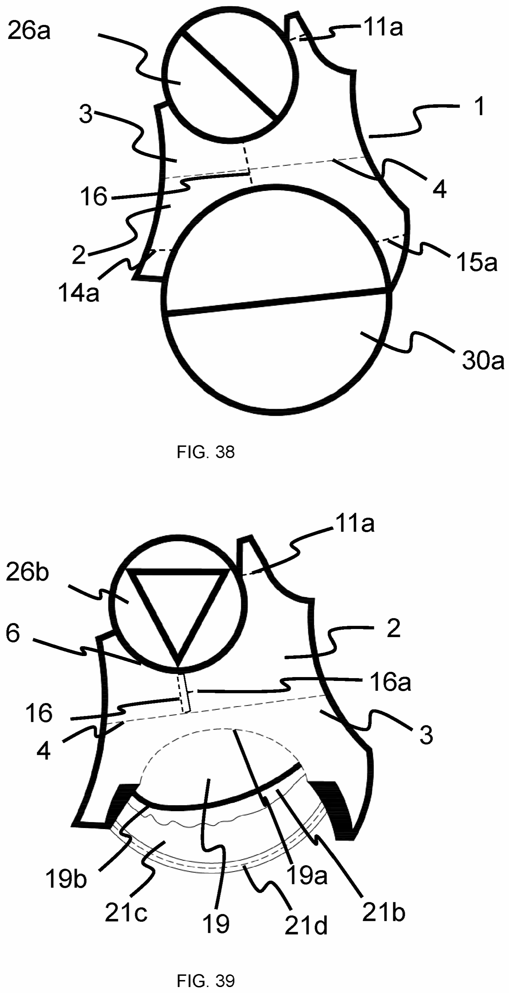

The drawing FIG. 38 shows the cardiac aorto-mitral prosthesis according to the claimed invention with the correct positioning of an attached artificial mechanical mitral valve and an attached artificial mechanical aortic valve in a view from above.

The drawing FIG. 39 shows the cardiac aorto-mitral prosthesis according to the claimed invention with the recess extension of the second leaflet recess for replacing the basis of the anterior leaflet of the innate mitral valve with an artificial biological aortic valve prosthesis in a virtual view from above.

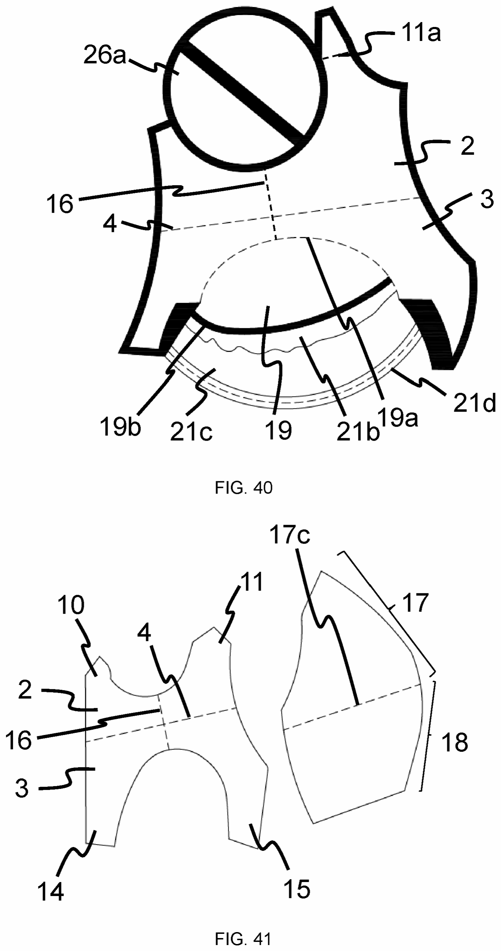

The drawing FIG. 40 shows the cardiac aorto-mitral prosthesis according to the claimed invention with the recess extension of the second leaflet recess for replacing the basis of the anterior leaflet of the innate mitral valve with an artificial mechanical aortic valve prosthesis.

The drawing FIG. 41 shows the cardiac aorto-mitral prosthesis according to the claimed invention in two leaflets and separated therefrom the combined third leaflet for the covering of the aorta wall and the fourth leaflet for replacing the left atrial roof for the production process in a view from above.

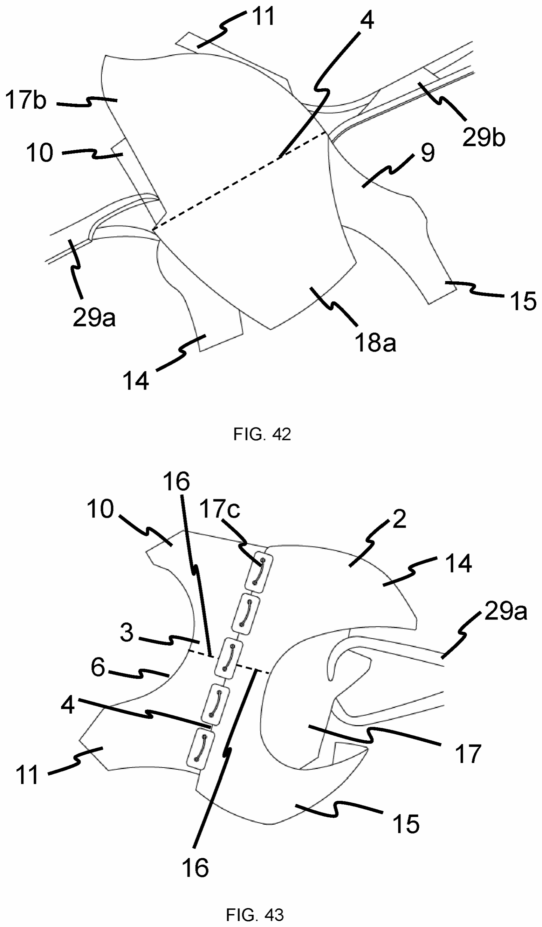

The drawing FIG. 42 shows the cardiac aorto-mitral prosthesis according to the claimed invention in two leaflets with the combined third leaflet and the fourth leaflet in the process of production in a view from above.

The drawing FIG. 43 shows the cardiac aorto-mitral prosthesis according to the claimed invention with four leaflets during the process of production, with the interrupted sutures placed at the center of the aorto-mitral curtain, separating the first leaflet from the second leaflet in the foreground, and separating the third leaflet from the fourth leaflet in the background in a view from below.

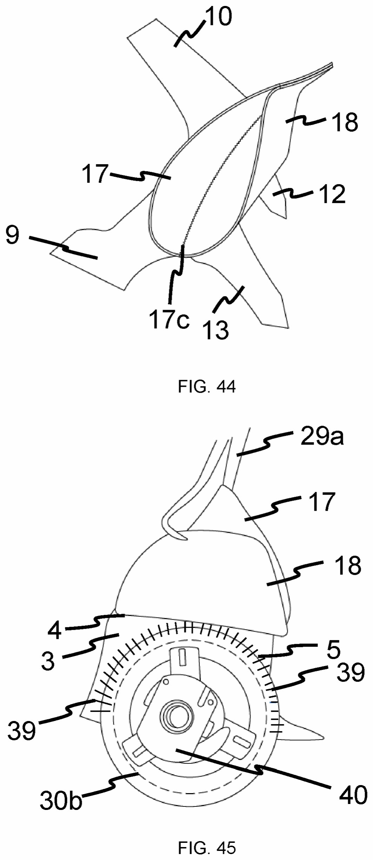

The drawing FIG. 44 shows the cardiac aorto-mitral prosthesis according to the claimed invention during the process of production, with a continuous suture placed at the center of the aorto-mitral curtain, separating the third leaflet from the fourth leaflet in the foreground, and separating the first leaflet form the second leaflet in the background in a view from above.

The drawing FIG. 45 shows the cardiac aorto-mitral prosthesis according to the claimed invention with four leaflets and an attached artificial biological mitral valve prosthesis that has been attached to the second leaflet, with the fourth leaflet in the foreground and the third leaflet in the background in a view from above.

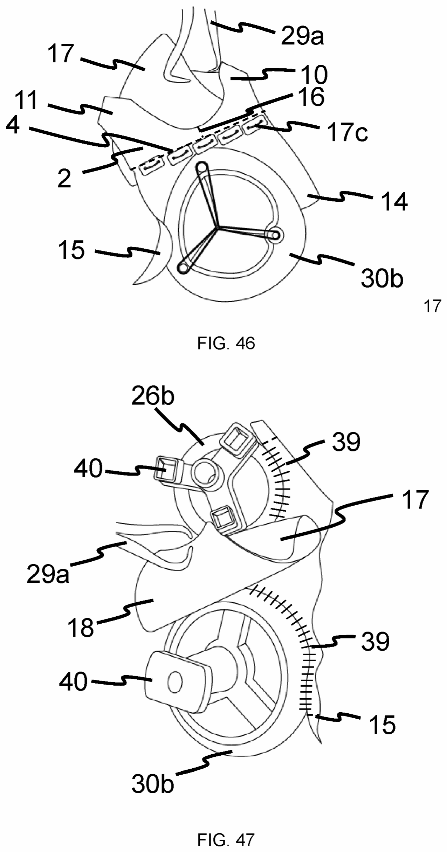

The drawing FIG. 46 shows the cardiac aorto-mitral prosthesis according to the claimed invention with four leaflets and an attached artificial biological mitral valve prosthesis attached to the second leaflet, with the first and second leaflet in the foreground and the third leaflet in the background in a virtual view from below.

The drawing FIG. 47 shows the cardiac aorto-mitral prosthesis according to the claimed invention with four leaflets and an artificial biological aortic valve attached to the first leaflet and an artificial biological mitral valve attached to the second leaflet, with an attached third leaflet and an attached fourth leaflet held in between a pair of tweezers in a virtual view from above.

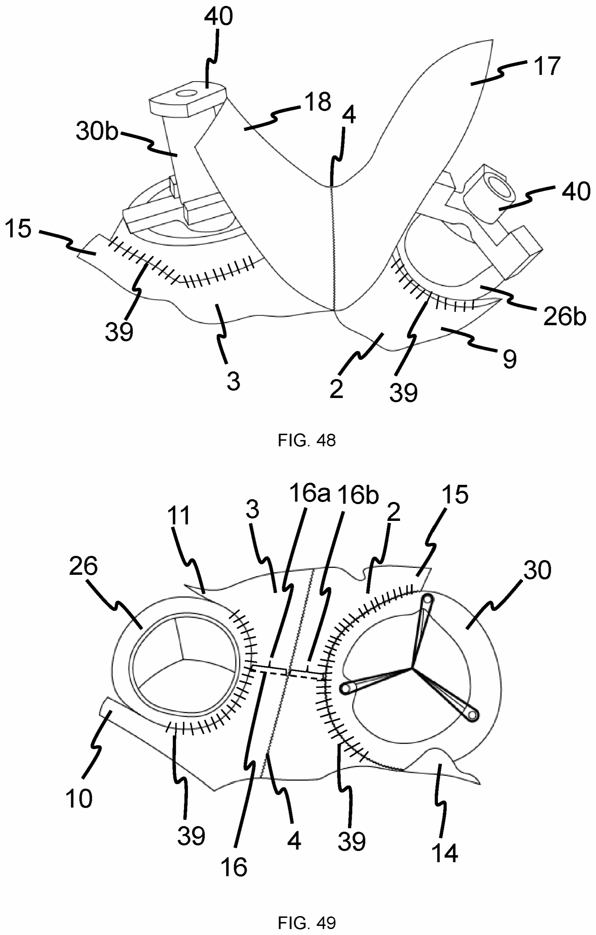

The drawing FIG. 48 shows the cardiac aorto-mitral prosthesis according to the claimed invention with four leaflets, an artificial biological aortic valve attached to the first leaflet and an artificial biological mitral valve attached to the second leaflet with the third leaflet and the fourth leaflet in a view from the side.

The drawing FIG. 49 shows the final prototype of a cardiac aorto-mitral prosthesis according to the claimed invention with four leaflets, an artificial biological aortic valve attached to the first leaflet and an artificial biological mitral valve attached to the second leaflet in a virtual view from below.

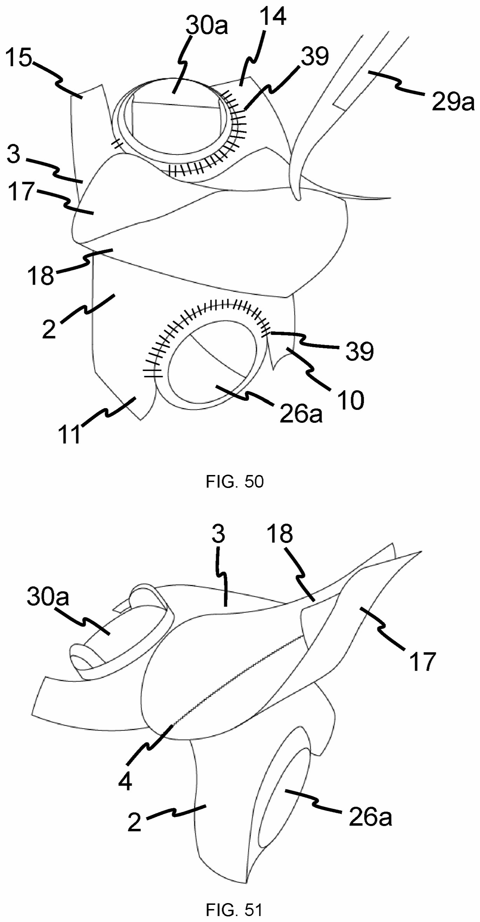

The drawing FIG. 50 shows the cardiac aorto-mitral prosthesis according to the claimed invention with four leaflets with an artificial mechanical aortic valve attached to the first leaflet and an artificial mechanical mitral valve attached to the second leaflet in a view from above.

The drawing FIG. 51 shows the cardiac aorto-mitral prosthesis according to the claimed invention with four leaflets, an artificial mechanical aortic valve attached to the first leaflet and an artificial mechanical mitral valve attached to the second leaflet in a view from the side.

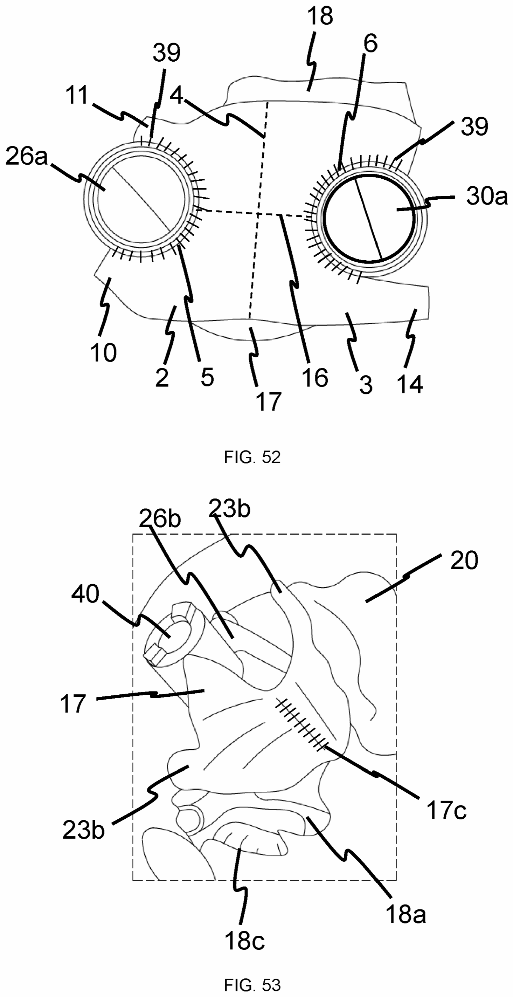

The drawing FIG. 52 shows the cardiac aorto-mitral prosthesis according to the claimed invention with four leaflets, an artificial mechanical aortic valve attached to the first leaflet and an artificial mechanical mitral valve attached to the second leaflet in a virtual view from below.

The drawing FIG. 53 shows the cardiac aorto-mitral prosthesis according to the claimed invention with four leaflets after the implantation after the aortic valve prosthesis has been implanted, when the third leaflet has not yet been sutured to the aortic wall and after the left atrial roof has been closed with the fourth leaflet in a view from above, as seen from outside of the heart during a sternotomy, when staying on the right side of the patient and looking towards the aortic root.

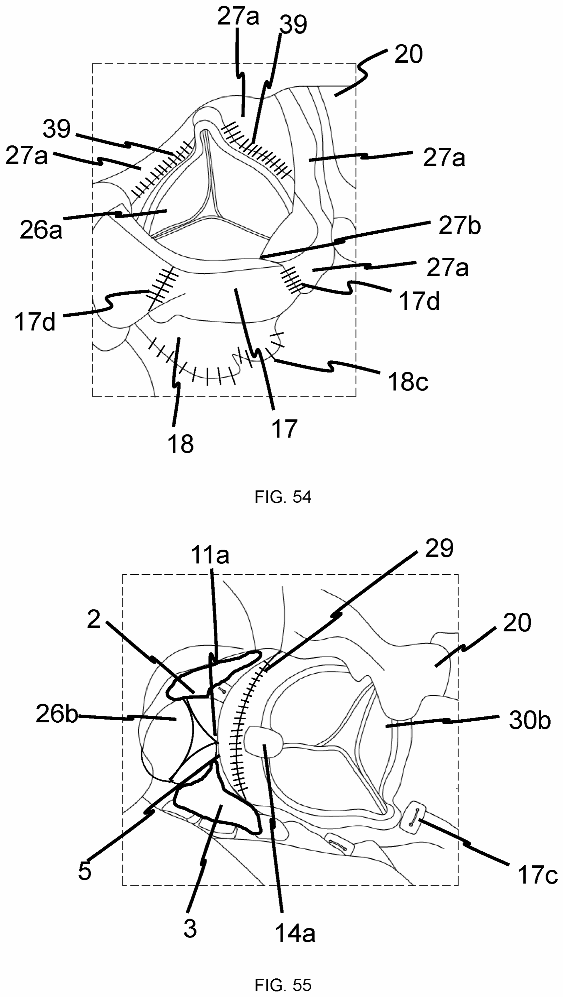

The drawing FIG. 54 shows the cardiac aorto-mitral prosthesis according to the claimed invention with four leaflets after the implantation of the aortic valve prosthesis with the second leaflet into the aorto-mitral curtain, when the third leaflet has not yet been sutured to the aortic wall and after the left atrial roof has been closed with the fourth leaflet and in a view from above as seen from outside of the heart, during a sternotomy, when staying on the right side of the patient and looking towards the aortic root.

The drawing FIG. 55 shows the cardiac aorto-mitral prosthesis according to the claimed invention with four leaflets and an artificial biological mitral valve prosthesis in the foreground and an artificial biological aortic valve prosthesis in the background attached to the first leaflet and the second leaflet of the cardiac aorto-mitral prosthesis, both having been implanted after a surgical implantation as seen from inside of the left ventricle when looking towards the aorto-mitral curtain.

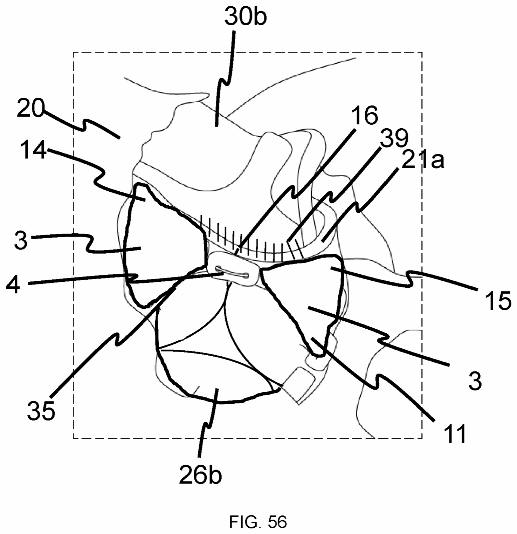

The drawing FIG. 56 shows the cardiac aorto-mitral prosthesis according to the claimed invention with four leaflets and an artificial biological mitral valve prosthesis and an artificial biological aortic valve prosthesis, both attached to the cardiac aorto-mitral prosthesis after surgical implantation as seen from inside of the left ventricular outflow tract, when looking towards the aorto-mitral curtain.

The drawing FIG. 1 shows the cardiac aorto-mitral prosthesis (**1**) with two leaflets (**2**,**3**) in a view from above, with a first leaflet **(2),** and a second leaflet **(3),** which are separated by a mental partition **(4)** that is a central line and that separates the prosthesis **(1)** into the first leaflet **(2)** and second leaflet **(3)** as two annular parts **(2,3)** between the aortic valve annulus and the mitral valve annulus of the human heart. The first leaflet **(2)** has a first leaflet recess **(5)** which serves for the inclusion of the aortic valve annulus, and the other second leaflet **(3)** has a second leaflet recess **(6)** which serves for the inclusion of the mitral valve annulus. The mental partition **(4)** may be marked onto the cardiac aorto-mitral prosthesis **(1)** for instance by a physiologically inert marker or by a simple folding. The prosthesis **(1)** has over both leaflets (**2**,**3**) one larger recess **(7)** for the support of the cardiac aorto-mitral prosthesis **(1)** to the tricuspid annulus and on the opposite side of the prosthesis **(1)** over both leaflets (**2**,**3**) an outer edge **(8)** which forms a support to the inner layer of the atrio-ventricular junction of the human heart. The cardiac aorto-mitral prosthesis **(1)** has on the first leaflet **(2)** two endings **(9,10).** On one ending **(9),** the cardiac aorto-mitral prosthesis **(1)** has a first leaflet extension **(11)** that extends the ending **(9)** to replace missing annular tissue of the commissure between the non-coronary and the right coronary cusp of the aortic valve. The first leaflet extension **(11)** may be adjusted as required during the surgical operation. The first leaflet extension **(11)** may be ending marked **(11a)** by a folding or by a visible line in separation from the ending **(9).** This helps alignment of the cardiac aorto-mitral prosthesis **(1)** during the surgical operation. The cardiac aorto-mitral prosthesis **(1)** has on the second leaflet **(3)** two endings **(12,13)** likewise. These endings **(12,13)** are extended by second leaflet extensions **(14,15).** The second leaflet extensions **(14,15)** are separated from the endings by ending markings **(14a,15a).** These ending markings **(14a,15a)** help alignment of the second leaflet extensions **(14,15)** to the antero-lateral commissure and the postero-medial commissure of the mitral valve during a surgical operation. The ending markings **(14a,15a)** may be imposed onto the cardiac aorto-mitral prosthesis (**1**) for instance by a physiologically inert marker, a suturing or by a simple folding.

The drawing FIG. 2 shows the cardiac aorto-mitral prosthesis **(1)** in two leaflets (**2**,**3**) in a view from below. The device **(1)** is the same as in the previous drawing, but turned by 180°. The mental partition **(4)** separates the two leaflets **(2,3).** A lengthwise mental partition **(16)** is present which separates the first **(2)** and the second **(3)** leaflet into two halves. The lengthwise mental partition **(16)** marks a connection from the commissure between left-coronary cusp and non-coronary cusp of the aortic valve to the center point of the anterior cusp of the mitral valve. This further helps alignment of the cardiac aorto-mitral prosthesis **(1)** during the surgical operation. The lengthwise mental partition **(16)** may also be combined with the mental partition **(4).** The drawing further shows the first leaflet **(2),** the second leaflet **(3),** the endings **(9,10,12,13),** the first leaflet recess **(5)** which serves for the inclusion of the aortic valve annulus, the second leaflet recess **(6)** which serves for the inclusion of the mitral valve annulus, the one larger recess **(7)** for the support to the tricuspid valve annulus and the outer edge **(8)** for the support to inner layer of the atrio-ventricular junction of the human heart.

The drawing FIG. 3 shows a third leaflet **(17)** which may be attached to the cardiac aorto-mitral prosthesis **(1)** along the mental partition **(4)** for an enlargement or a replacement of the wall of the aortic root and aorta. The attachment of the third leaflet **(17)** to the mental partition **(4)** of the prosthesis **(1)** is carried out along the straight cutting **(17a)** of the third leaflet pericardial patch **(17b).**

The drawing FIG. 4 shows the third leaflet **(17)** to which a fourth leaflet **(18)** has been attached. The fourth leaflet **(18)** with the fourth leaflet pericardial patch **(18a)** has been attached to the straight cutting **(17a)** of the third leaflet **(17).** The straight cutting **(17a)** is then attached by a suturing to the mental partition **(4)** of the cardiac aorto-mitral prosthesis **(1).** The fourth leaflet **(18)** serves for the enlargement or closure of the left atrial roof at the location of the atrial incision.

The drawing FIG. 5 shows the third leaflet **(17)** to which a fourth leaflet has been attached. The pericardial patch is the same as in the drawing FIG. 4, but turned by 180°. The third leaflet patch area **(17b)** is used for the enlargement or replacement of the wall of the aortic root and aorta, the fourth leaflet patch area **(18a)** is used for a replacement or closure of the left atrial roof at the location of the atrial incision. Along the straight cutting **(17a)** of the third leaflet **(3),** the fourth leaflet pericardial patch **(18a)** has been attached. On the other side of the straight cutting **(17a)** between the third leaflet **(17)** and fourth leaflet **(18),** the mental partition **(4)** between the first leaflet **(2)** and the second leaflet **(3)** is situated.

The drawing FIG. 6 shows the cardiac aorto-mitral prosthesis **(1)** that has a recess extension **(19)** that extends over the recess ending **(19a)** which marks the anterior mitral valve annulus. The recess ending **(19a)** without the extension **(19)** forms the recess **(6)** which serves for the inclusion of the mitral valve annulus. This recess extension **(19)** may be used during a surgical operation to replace proceeding damage into the anterior mitral valve annulus so that the natural mitral valve coaptation is preserved. The recess extension **(19)** may be adapted in its exact size by curtailing shortly before the surgical operation, when the human heart has been opened and the extent of damage to the anterior leaflet of the mitral valve is visible. The curtailing may be accomplished for instance by surgical scissors. The recess extension **(19)** then replaces the part of the mitral valve and the part of mitral valve annulus that have been damaged, by suturing the recess extension boundary **(19b)** of the recess extension **(19)** to the remaining part of the anterior annulus of the mitral valve. The recess extension **(19)** thus saves time during a surgical operation and allows the reconstruction of the mitral valve without unnecessary replacement. The cardiac aorto-mitral prosthesis **(1)** with the recess extension **(19)** is also called a "hemi-conduit".

The drawing FIG. 7 shows the cardiac aorto-mitral prosthesis **(1)** that has a recess extension **(19)** of the recess ending **(19a)** which serves for the inclusion of the mitral valve annulus, turned by 180° against the previous drawing. The cardiac aorto-mitral prosthesis **(1)** shows the endings **(9,10,12,13),** the recess **(5)** which serves for the inclusion of the aortic valve annulus, the recess **(6)** which serves for the inclusion of the mitral valve annulus, the one larger recess **(7)** for the support to the tricuspid annulus and the outer edge **(8)** which forms a support to the frame of the inner layer of the atrio-ventricular junction of the human heart. The mental partition **(4)** and the lengthwise mental partition **(16)** are visible. The mental partition **(4)** separates the first leaflet **(2)** and the second leaflet **(3)** and the lengthwise central partition **(16)** separates the halves of the first leaflet **(2)** and the second leaflet **(3).** The lengthwise mental partition **(16)** further aligns the cardiac aorto-mitral prosthesis **(1)** and helps the surgeon for a proper alignment of the cardiac aorto-mitral prosthesis **(1).** The recess extension **(19)** which may be used for replacing a part of the mitral valve and which serves at the recess ending **(19a)** for the inclusion of the mitral valve annulus is seen.

The drawing FIG. 8 shows the cardiac aorto-mitral prosthesis **(1)** with the third leaflet pericardial patch **(17b)** for a replacement or enlargement of the wall of the aortic root and aorta wall and the fourth leaflet pericardial patch **(18a)** for the reconstruction or closure of the left atrial roof in the foreground with the first leaflet **(2)** and the second leaflet **(3)** in the background. The endings (**9**,**10**) of the first leaflet **(2)** and the endings **(12,13)** of the second leaflet **(3)** can be seen. The third leaflet **(17)** and the fourth leaflet **(18)** are sutured to each other on the straight cutting **(17a)** of the third leaflet pericardial patch **(17b).** The line of the straight cutting **(17a)** concurs with the mental partition **(4)** that separates the first leaflet **(2)** and the second leaflet **(3).** By the cardiac aorto-mitral prosthesis **(1)** with the third leaflet pericardial patch **(17b)** and the fourth leaflet pericardial patch **(18a),** the wall of the aorta and aortic root can be covered and the left atrial roof can be reconstructed with a replacement of the aorto-mitral curtain by the first leaflet **(2)** and the second leaflet **(3).** The suture at the straight cutting **(17a)** over the mental partition **(4)** merges all four leaflets at the center line of the cardiac aorto-mitral prosthesis **(1).**

The drawing FIG. 9 shows the cardiac aorto-mitral prosthesis **(1)** with the first leaflet **(2)** and the second leaflet **(3)** in the foreground and with the third leaflet pericardial patch **(17b)** and the fourth leaflet pericardial patch **(18a)** in the background. The second leaflet **(3)** has a recess extension **(19).** By the recess extension, a damaged part of the mitral valve and proceeding damage into the anterior mitral valve annulus may be replaced. The mental partition **(4)** between the first leaflet **(2)** and the second leaflet **(3)** is also shown in the foreground. On the other side of the mental partition **(4),** the third leaflet **(17)** and the fourth leaflet **(18)** are attached to the first **(2)** and the second leaflet **(3)** by a suturing along the straight cutting **(17a).**

The drawing FIG. 10 shows the second leaflet **(3)** with the recess extension **(19)** attached to the second leaflet **(3).** The mental partition **(4)** which separates the second leaflet **(3)** from the first leaflet **(2)** and the straight cutting **(17a)** which is sutured to the mental partition **(4)** and the fourth leaflet pericardial patch **(18a)** are also shown. The second leaflet **(3)** also has as lengthwise mental partition **(16)** which gives the surgeon orientation by keeping in line with the commissures of the aortic valve anterior cusp of the mitral valve. This saves time and increases the precision during the surgical operation.

The drawing FIG. 11 shows the second leaflet **(3)** with an attached fourth leaflet **(18)** with the fourth leaflet pericardial patch **(18a),** turned by 180° against the previous drawing. In this embodiment, the first **(2)** and the second leaflet **(3)** serve for the purpose that has been described for the first **(2)** and the second leaflet **(3),** and the fourth leaflet **(18)** serves for the purpose that has been described for the fourth leaflet **(18).**

The drawing FIG. 12 shows the second leaflet **(3)** with an attached fourth leaflet **(18)** with the fourth leaflet pericardial patch **(18a).** The mental partition **(4)** which separates the first leaflet **(2)** and the second leaflet **(3),** the straight cutting **(17a)** which is sutured to the mental partition **(4)** and the further lengthwise mental partition **(16)** are visible. The recess extension **(19)** of the recess ending **(19a)** which serves for the inclusion of the mitral valve annulus was present and has been completely cut out along the recess ending **(19a).** The cutting of the recess extension **(19)** is carried out when the mitral valve annulus of the human heart after the opening of the heart shows to be in good order so that a replacement of the part of the mitral valve or the mitral valve annulus is not necessary.

The drawing FIG. 13 shows the second leaflet **(3)** with an attached fourth leaflet **(18)** as a two-leaflet aorto-mitral prosthesis, turned by 180° against the previous drawing. The recess extension **(19)** of the recess ending **(19a)** was present and has been cut out, as the mitral valve has shown to be in good order along the recess ending **(19a)** for the surgical operation.

The drawing FIG. 14 shows the fourth leaflet **(18)** which serves for the replacement of the left atrial roof with the fourth leaflet pericardial patch **(18a)** and a fourth leaflet straight cutting **(18b)** of the fourth leaflet pericardial patch **(18a).** The fourth leaflet **(18)** has been attached to the second leaflet **(3)** and the first leaflet **(2)** which is not visible here. The attachment has been carried out by a suturing along the fourth leaflet straight cutting **(18b)** of the fourth leaflet **(18)** and the mental partition **(4)** between the first **(2)** and the second leaflet **(3).** The endings **(12,13)** of the second leaflet **(3)** are visible.

The drawing FIG. 15 shows the third leaflet pericardial patch **(17a)** which has been attached to a second leaflet **(3).** The first leaflet **(2)** is not visible here. The attachment has been carried out along the mental partition **(4)** between the first leaflet **(2)** and the second leaflet **(3)** and the straight cutting **(17b)** of the third pericardial patch **(17).** The second leaflet **(3)** has a recess extension **(19)** which serves for the partial replacement of the anterior cusp of the mitral valve to achieve valve reconstruction and avoid the replacement of the mitral valve.

The drawing FIG. 16 shows a third leaflet pericardial patch **(17b)** with an attached fourth leaflet pericardial patch **(18a)** in the foreground. A first **(2)** and a second leaflet **(3)** have also been attached to the combined third **(17)** and fourth leaflet **(18).** The endings **(12,13)** of the second leaflet **(3)** are visible in the background. The first leaflet **(2)** is not visible here. This embodiment is indicated when an aortic wall replacement with the third leaflet **(17)** or a replacement of the left atrial roof with the fourth leaflet **(18),** as described above, is implemented.

The drawing FIG. 17 shows a third leaflet pericardial patch **(17b)** and a fourth leaflet pericardial patch **(18a)** in the foreground. Along the straight cutting **(17a)** of the third leaflet pericardial patch **(17b)** on the other side, a second leaflet **(3)** has been attached. The endings **(12,13)** of the second leaflet **(3)** are visible. The first leaflet **(2)** is not visible here. This embodiment is indicated when an aortic wall replacement with the third leaflet **(17),** as described above, is implemented.

The drawing FIG. 18 shows a drawing of the human heart **(20)** with the anatomic features that are relevant for the use of the invention. The drawing shows the mitral valve **(21),** the tricuspid valve **(22),** the aortic valve **(23)** and the pulmonary valve **(24).** Between the mitral valve **(21)** and the aortic valve **(23),** the aorto-mitral curtain **(25)** is situated. In the aorto-mitral curtain **(25),** the left trigone **(25a)** and the right trigone **(25b)** are situated. The aorto-mitral curtain **(25)** may be subject to damages, for instance by inflammation after a germ ingress. It may also be possible that the aorto-mitral curtain **(25)** shows damage during another heart operation, for instance a valve replacement operation. In this case, a replacement of the aorto-mitral curtain **(25)** must be carried out. The cardiac aorto-mitral prosthesis **(1)** of the current patent application is able to replace the aorto-mitral curtain **(25)** without a lengthy and risky procedure with a pericardial patch during a surgical operation to the human heart. The mitral valve **(21)** and the tricuspid valve **(22)** are surrounded by the mitral valve annulus **(21a)** and the tricuspid valve annulus **(22a).**

The drawing FIG. 19 shows the position of the cardiac aorto-mitral prosthesis **(1)** of the current invention for the replacement of the intervalvular fibrous body. The cardiac aorto-mitral prosthesis **(1)** which is a pericardial patch replaces the entire aorto-mitral curtain **(25),** including the left fibrous trigone **(25a),** the right fibrous trigone **(25b)** and the central part **(25c)** of the cardiac aorto-mitral prosthesis **(1).** In the cardiac aorto-mitral prosthesis **(1),** the first leaflet and the endings **(12,13)** of the second leaflet **(3)** of the cardiac aorto-mitral prosthesis **(1)** can be seen. By the shape of the aorto-mitral prosthesis **(1),** the replacement may be carried out quickly and without risks. The aorto-mitral prosthesis **(1)** has been prepared from a pericardial patch preferably before the surgical operation.

The drawing FIG. 20 shows the cardiac aorto-mitral prosthesis **(1)** of the present invention with four leaflets **(2,3,17,18)** during implantation. The suture of the first **(2)** and second leaflet **(3)** to the human heart **(20)** has been completed. The first leaflet **(2)** and the second leaflet **(3)** are not visible here. To the cardiac aorto-mitral prosthesis **(1),** a third leaflet **(17)** and a fourth leaflet **(18)** have been attached. The third leaflet pericardial patch **(17b)** and the fourth leaflet pericardial patch **(18a)** can be seen. To the cardiac aorto-mitral prosthesis **(1),** an artificial mechanical aortic valve prosthesis **(26a)** has been attached. The third leaflet pericardial patch **(17b)** for the replacement of the aortic wall **(27a)** and to cover the ascending aorta **(27)** can be seen. The fourth leaflet pericardial patch **(18a)** for the enlargement of the left atrial roof **(28)** can also be seen. The cardiac aorto-mitral prosthesis **(1)** with the artificial mechanical aortic valve prosthesis **(26a)** is inserted into the human heart **(20)** by an aortic wall incision **(27b).** The third leaflet pericardial patch **(17b)** is brought close to the aorta **(27)** to replace the aortic wall **(27a)** and its aortic wall incision **(27b).** The third leaflet pericardial patch **(17b)** is lifted by a pair of tweezers **(29a).** The aortic wall **(27a)** is lifted away from the tissue around the aorta **(27c)** by another pair of tweezers **(29b).**

The drawing FIG. 21 shows the cardiac aorto-mitral prosthesis **(1)** with a third leaflet **(17)** and a fourth leaflet **(18)** during implantation. The third leaflet pericardial patch **(17b)** and the fourth leaflet pericardial patch **(18a)** are visible. After the suture of the first leaflet **(2)** and of the second leaflet **(3)** as a cardiac aorto-mitral prosthesis **(1)** has been completed, the left atrial roof **(28)** is replaced with the fourth leaflet pericardial patch **(18a).** The fourth leaflet sutures **(18c)** of the fourth leaflet pericardial patch **(18a)** to the left atrial roof **(28)** are visible. The third leaflet pericardial patch **(17b)** is prepared for closing the aortic wall **(27a).** The cardiac aorto-mitral prosthesis **(1)** is inserted with an artificial mechanical mitral valve prosthesis **(30a).** The artificial mechanical mitral valve prosthesis **(30a)** is visible through the aortic valve annulus **(23a).** An artificial aortic valve prosthesis **(26)** (not visible) is prepared for insertion. The sutures **(31)** to the aortic valve annulus **(23a)** are prepared for the implantation of the artificial aortic valve prosthesis **(26).** The left coronary ostium **(32a)** and the right coronary ostium **(32b)** are visible in the wall of the aortic root **(23b)** during the surgical operation.

The drawing FIG. 22 shows the human heart **(20)** with the innate left trigone **(25a),** and the innate right trigone **(25b)** during the replacement of the central part of the aorto-mitral curtain **(25c)** that is prepared to be closed with a cardiac aorto-mitral prosthesis **(1).** An artificial aortic valve prosthesis **(26),** which is not seen here, will be positioned through an aortic wall incision **(27b)** for replacement, during a surgical operation on the aortic valve **(21)** and on the aorto-mitral curtain **(25).** The aortic wall **(27a)** is kept away from the aortic valve **(21)** by a pair of tweezers **(29a).** Further instruments **(33)** which are used during the surgical operation are also shown.

The drawing FIG. 23 shows the human heart **(20)** with an aortic wall incision **(27b).** The third leaflet pericardial patch **(17b)** of the cardiac aorto-mitral prosthesis **(1)** is seen that is prepared for a replacement of the aortic wall **(27a)** during a surgical operation. The fourth leaflet pericardial patch **(18a)** of the cardiac aorto-mitral prosthesis **(1)** is seen for closing the left atrial roof **(28).** The replacement of the aorto-mitral curtain **(25)** with the cardiac aorto-mitral prosthesis **(1)** is not seen here. Further instruments **(33)** which are used during the surgical operation are also shown. The innate aortic valve **(23)** is also seen.

The drawing FIG. 24 shows the human heart **(20)** with the left trigone **(25a)** and the right trigone **(25b)** for a replacement of the intervalvular fibrous body by the cardiac aorto-mitral prosthesis **(1)** between the aortic valve annulus **(23a)** and the mitral valve annulus **(21a).** The fourth leaflet pericardial patch **(18a)** is replacing the left atrial roof **(28).** A aorta tube prosthesis with valve **(34)** to replace the ascending aorta **(27),** as described by Bentall et al., has been sutured to the cardiac aorto-mitral prosthesis **(1).** The aorta tube prosthesis with valve **(34)** for the ascending aorta **(27)** and with it the cardiac aorto-mitral prosthesis **(1)** and the fourth leaflet **(18)** is lifted by a pair of tweezers **(29a).** The aortic root **(23b)** and the aortic wall **(27a)** are shown. Further instruments **(33)** which are used during the surgical operation are also shown.

The drawing FIG. 25 shows an X-ray photography of the human heart **(20)** with an artificial mechanical aortic valve prosthesis **(26a)** and an artificial mechanical mitral valve prosthesis **(30a).** In between the artificial valves **(26a,30a),** the aorto-mitral angle α (**35**) is seen in a side view. The cardiac aorto-mitral prosthesis **(1)** of the current invention perfectly adapts to the aorto-mitral angle α **(35)** by its flexibility.

The drawing FIG. 26 shows an X-ray photography of the human heart **(20)** in the human body in a closer detail. A visible artificial biological aortic valve prosthesis **(26b)** and a visible artificial biological mitral valve prosthesis **(30b)** can be seen. In the aorto-mitral angle **(35),** the intervalvular fibrous body of the aorto-mitral curtain **(25)** is situated. The aortic wall **(27a)** and the left atrial roof **(28)** can be seen.

The drawing FIG. 27 shows the cardiac aorto-mitral prosthesis **(1)** with four leaflets. To the first leaflet **(2)** and the second leaflet **(3),** a third leaflet pericardial patch **(17b)** and a fourth leaflet pericardial patch **(18a)** are attached. The cardiac aorto-mitral prosthesis **(1)** is shown as used for implantation as a cardiac aorto-mitral prosthesis **(1).** The first **(2)** and the second leaflet **(3)** are attached to each other at the mental partition **(4)** between the first **(2)** and the second leaflet **(3).** The first **(2)** and the second leaflet **(3)** form the cardiac aorto-mitral prosthesis **(1).** The endings **(9,10)** of the first leaflet **(2)** and the endings (**12**,**13**) of the second leaflet **(3)** can be seen. A third leaflet **(17)** in the form of a third leaflet pericardial patch **(17b)** and a fourth leaflet **(18)** in the form of a fourth leaflet pericardial patch **(18a)** are attached to the cardiac aorto-mitral prosthesis **(1).** Between the first leaflet **(2)** and the second leaflet **(3),** a stretching angle β **(35a)** opens which is placed during the surgical operation into the aorto-mitral angle α **(35).** The first leaflet **(2)** and the second leaflet **(3)** are placed during replacement according to the invention in between the mitral valve **(21)** and the aortic valve **(23)** of the human heart **(20)** and into the aorto-mitral angle α **(35)** between the mitral valve **(21)** and the aortic valve **(23)** with the intervalvular fibrous body.

The drawing FIG. 28 shows the cardiac aorto-mitral prosthesis **(1)** with the first leaflet **(2)** and the second leaflet **(3),** an attached third leaflet pericardial patch **(17b)** and an attached fourth leaflet pericardial patch **(18a)** in between two fingers **(36).** The cardiac aorto-mitral prosthesis **(1)** is kept on the fourth leaflet pericardial patch **(18a)** in between two fingers **(36).** The endings (**12**,**13**) of the second leaflet **(3)** and the first leaflet extension **(11)** are visible. The first leaflet **(2)** and the second leaflet **(3)** serve for the replacement of the intervalvular fibrous body.

The drawing FIG. 29 shows the cardiac aorto-mitral prosthesis **(1)** with the first leaflet **(2)** and the second leaflet **(3),** an attached third leaflet pericardial patch **(17b)** and an attached fourth leaflet pericardial patch **(18a)** in between two fingers **(36),** and the stretching angle β **(35a)** that opens up between the first leaflet **(2)** and the second leaflet **(3).** The stretching angle β **(35a)** is able to match the aorto-mitral angle α **(35)** between the mitral valve **(21)** and the aortic valve **(23)** due to the elasticity of the cardiac aorto-mitral prosthesis **(1).**

The drawing FIG. 30 shows the aortic wall incision **(27b)** into the aortic wall **(27a)** and the aortic root **(23b)** of the aorta **(27)** of the human heart **(20)** with an implanted cardiac aorto-mitral prosthesis **(1)** after the implantation of the first leaflet **(2)** and the second leaflet **(3)** has been completed. The aorta has been shut off by a pair of clamps **(38).** An artificial biological mitral valve prosthesis **(30b)** has been positioned and sutured to the human heart **(20).** The incision in the left atrial roof **(28)** has been closed by suturing the fourth leaflet **(18)** with fourth leaflet sutures **(18c)** to the left atrial roof **(28).** The third leaflet pericardial patch **(17b)** for the replacement of the aortic wall **(27a)** is partially sutured by third leaflet sutures **(17c)** and is prepared for replacing the aortic wall **(27a).** The aorta **(27)** has been opened by an aortic wall incision **(27b)** and through the aortic wall incision **(27b)** and after the removal of the innate aortic valve **(23),** the left ventricular outflow tract **(37)** is visible. In this constellation, the aortic wall **(27a)** will be fully closed by the third leaflet pericardial patch **(17b)** of the cardiac aorto-mitral prosthesis **(1),** and the surgical operation will then be complete. The clamps **(38)** can be seen that hold and shut off the ascending aorta **(27).**

The drawing FIG. 31 shows the aortic wall incision **(27b)** into the aortic wall **(27a)** during the implantation of a cardiac aorto-mitral prosthesis **(1)** with four leaflets. After the suture of the first leaflet **(2)** and second leaflet **(3)** has been completed, the artificial mitral valve prosthesis **(30a)** has been positioned and sutured. The aortic wall incision **(27b)** will be closed by a suturing of the fourth leaflet pericardial patch **(18)** into the aortic wall incision **(27b).** For the replacement of the aortic wall **(27a),** the third leaflet **(17b)** is prepared for suture and an artificial mechanical aortic valve prosthesis **(30a)** is positioned into the aortic valve annulus **(23a)** for replacement. The fourth leaflet pericardial patch **(18a)** which is prepared for replacing the left atrial roof **(28)** is shown.

The drawing FIG. 32 shows the ascending aorta **(27)** of the human heart **(20)** during the last step of implantation of the cardiac aorto-mitral prosthesis **(1)** of the aortic wall **(27a).** The aortic wall incision **(27b)** into the aortic wall **(27a)** has been closed by the third leaflet **(17)** with its pericardial patch **(17b).** The third leaflet **(17)** is attached to the cardiac aorto-mitral prosthesis **(1)** which is not visible here.

The drawing FIG. 33 shows the cardiac aorto-mitral prosthesis **(1),** as described in the drawing FIG. 1, with an attached artificial biological mitral valve prosthesis **(30b).** By this embodiment, the cardiac aorto-mitral prosthesis **(1)** may be used for a replacement of the intervalvular fibrous body with a replacement of the innate mitral valve **(21).** The mental partition length **(4a)** of the mental partition **(4)** of the cardiac aorto-mitral prosthesis **(1)** is 26 mm to 34 mm, depending on the size of the artificial biological mitral valve prosthesis **(30b)** and the second leaflet recess **(6).** The size and the dimensions of the cardiac aorto-mitral prosthesis **(1)** may be adapted during the surgical operation depending on the individual anatomical features of each patient, for instance by cutting. The position of the lengthwise mental partition **(16)** may be used to align the cardiac aorto-mitral prosthesis **(1)** during the surgical operation to the antero-lateral commissure and the postero-medial commissure of the artificial biological mitral valve prosthesis **(30b).** After the attachment of the artificial biological mitral valve prosthesis **(30b)** to the cardiac aorto-mitral prosthesis **(1),** the distance (**16a**) from the second leaflet recess **(6)** to the mental line **(4)** is in a typical embodiment 1 mm. The mental partition ending markings **(14a,15b)** of the second leaflet extensions **(14,15)** may be used to align the cardiac aorto-mitral prosthesis **(1)** to the valve sutures **(39)** of the artificial biological mitral valve prosthesis **(30b)** to the cardiac aorto-mitral prosthesis **(1)** during the surgical operation.

The drawing FIG. 34 shows the cardiac aorto-mitral prosthesis **(1),** as described in the drawing FIG. 1, with an attached artificial mechanical mitral valve prosthesis **(30a).** The embodiment is identical to the embodiment that was described in FIG. 33 except for that the artificial biological mitral valve **(30b)** is an artificial mechanical mitral valve **(30a).**

The drawing FIG. 35 shows the cardiac aorto-mitral prosthesis **(1)** with four leaflets and an attached artificial biological mitral valve prosthesis **(30b),** in a view from below. The mental partition length **(4a)** of the mental partition **(4)** of the cardiac aorto-mitral prosthesis **(1)** is 26 mm to 34 mm, depending on the size of the artificial mitral valve prosthesis **(30).** The size and the dimensions of the cardiac aorto-mitral prosthesis **(1)** may be adapted during the surgical operation depending on the individual anatomical features of each patient, for instance by cutting. After the attachment of the artificial biological mitral valve prosthesis **(30b)** to the cardiac aorto-mitral prosthesis **(1),** the distance **(16a)** from the second leaflet recess **(6)** to the mental line **(4)** is in a typical embodiment 1 mm. The ending markings **(14a,15b)** of the second leaflet extensions **(14,15)** may be used to align the cardiac aorto-mitral prosthesis **(1)** to the sutures of the mitral valve **(21)** to the cardiac aorto-mitral prosthesis **(1)** during the surgical operation. The third leaflet **(17)** and the fourth leaflet **(18)** are visible in the background. The mental partition **(4)** is the line of suture for the attachment of the third leaflet pericardial patch **(17b)** and the fourth leaflet pericardial patch **(18a).**

The drawing FIG. 36 shows the cardiac aorto-mitral prosthesis **(1)** with four leaflets and an attached mechanical artificial mitral valve prosthesis **(34b).** The embodiment is identical to the embodiment that was described in FIG. 35 except that a mechanical artificial mitral valve prosthesis **(30a)** is used instead of an artificial biological mitral valve prosthesis **(30b).** As an artificial mechanical mitral valve prosthesis **(30a)** is used in this embodiment, the lengthwise mental partition **(16)** may be omitted. The third leaflet **(17)** and the fourth leaflet **(18)** are visible in the background.

The drawing FIG. 37 shows the cardiac aorto-mitral prosthesis **(1)** with an attached artificial biological mitral valve prosthesis **(30b)** and an attached artificial biological aortic valve prosthesis **(26b).** In this embodiment, the aorto-mitral curtain **(25)** with left trigon **(25a)** and the right trigon **(25b)** may be replaced with a cardiac aorto-mitral prosthesis **(1)** that comprises an artificial biological mitral valve prosthesis **(30b)** and an artificial biological aortic valve prosthesis **(26b)** for the replacement of the innate mitral valve **(21)** and the innate aortic valve prosthesis **(23).** In the cardiac aorto-mitral prosthesis **(1),** the lengthwise mental partition **(16)** is visible. The lengthwise mental partition **(16)** may be used to properly align the commissures of the artificial biological mitral valve prosthesis **(30b)** and an artificial biological aortic valve prosthesis **(26b).** To the cardiac aorto-mitral prosthesis **(1),** ending markings **(11a,14a,15a)** have been applied. The ending markings **(11a, 14a, 15a)** correspond to the commissures of the artificial biological mitral valve prosthesis **(30b)** and of the artificial biological aortic valve prosthesis **(26b).** One of the commissures of the artificial biological mitral valve prosthesis **(30b)** is placed in such a way that it corresponds to the center of the second leaflet recess **(6)** and one of the ending markings **(11a)** is placed in such a way that it corresponds in the artificial biological aortic valve prosthesis **(26b)** to the commissure between the left-coronary and the right coronary cusp, the commissure between the right coronary cusp and the non-coronary cusp and the nadir of the left coronary commissure of an innate aortic valve **(23)** below the left coronary ostium **(32a).** This is crucial to establish the later hemodynamic properties of the mitral and aortic valves in the left ventricular outflow tract **(37),** and thus for the patient to recover. The lengthwise mental partition **(16)** may be used to properly align one of the commissures of the artificial biological mitral valve prosthesis **(30b)** and of the artificial biological aortic valve prosthesis **(26b)** and helps to properly place the artificial valves with the cardiac aorto-mitral prosthesis **(1).**

The drawing FIG. 38 shows the cardiac aorto-mitral prosthesis **(1)** with an attached artificial mechanical mitral valve **(30a)** and an attached artificial mechanical aortic valve prosthesis **(26a).** Other than for a biological prosthesis, the placement of the commissures of the artificial mechanical aortic valve prosthesis **(30a)** does not correspond to the commissure between the left-coronary and the right coronary cusp, the commissure between the right coronary and the non-coronary cusp and the nadir of the left coronary commissure of an innate aortic valve **(23).** The center of one of the cusps of the artificial mechanical aortic valve prosthesis **(26a)** corresponds to the nadir of the left coronary commissure, below the left coronary ostium **(32a).** The commissures of the artificial mechanical mitral valve prosthesis **(30a)** may be placed equidistantly to the ending markings **(14a,15a)** of the second leaflet **(3).** The center of the cusp of the artificial mechanical mitral valve prosthesis **(1)** then corresponds to the center of the anterior mitral valve annulus **(21a).** This relation is crucial to re-establish the innate hemodynamic properties of the mitral and aortic valves in the left ventricular outflow tract **(35b),** and thus for the patient to recover.

The drawing FIG. 39 shows the cardiac aorto-mitral prosthesis **(1)** with an attached artificial biological aortic valve prosthesis **(26b)** and a recess extension **(19).** The surgical operation that is carried out with this embodiment is also called the "hemi-Commando"-operation. In the surgical operation, the recess ending **(19a)** of the recess extension **(19)** is replacing the annular portion of the anterior cusp **(21b)** of the mitral valve **(21)** and is sutured by a continuous suture to the residual tissue of the anterior cusp **(21b)** of the innate mitral valve **(21).** Thus, the anterior cusp **(21b)** of the mitral valve annulus **(21a)** is reconstructed and the part of the mitral valve **(21)** that has been damaged by inflammation in the range of the recess extension **(19)** to the recess extension boundary **(19b)** may be removed. The distance **(16a)** from the second leaflet recess **(6)** to the mental line **(4)** of the cardiac aorto-mitral curtain prosthesis **(1)** is in a typical embodiment 1 mm after the replacement of the cardiac aorto-mitral curtain prosthesis **(1).** The posterior cusp **(21c)** of the innate mitral valve **(21)** which has not been damaged by inflammation may be reinforced by an annuloplasty band **(21d).** Thus, the mitral valve **(21)** is reconstructed and the innate coaptation line of the mitral valve cusps is maintained, as well as the innate subvalvular apparatus. The placement of the commissures of the artificial biological aortic valve prosthesis **(26b)** corresponds to the commissure between the left-coronary cusp and the right coronary cusp, the commissure between the right coronary and the non-coronary cusp and the nadir of the left coronary commissure and the ending marking **(11a)** on the first leaflet **(2),** below the left coronary ostium **(32a).** In this embodiment, the cardiac aorto-mitral prosthesis **(1)** may also be provided with a third leaflet **(17)** or a fourth leaflet **(18)** or both a third leaflet **(17)** and a fourth leaflet **(18)** on the other side of the mental partition **(4).**

The drawing FIG. 40 shows the cardiac aorto-mitral hemi-prosthesis **(1)** with an attached artificial mechanical aortic valve prosthesis **(26a)** and a recess extension **(19).** The drawing shows the same embodiment as the drawing FIG. 39, with an attached artificial mechanical aortic valve prosthesis **(26a)** instead of an attached artificial biological aortic valve prosthesis **(26b).** The ending marking **(11a)** of the first leaflet does not correspond to the commissure between the left-coronary cusp and the right coronary cusp, the commissure between the right coronary cusp and the non-coronary cusp and the nadir of the left coronary commissure of the artificial mechanical aortic valve prosthesis **(26a).** However, the center of one of the cusps of the artificial mechanical aortic valve prosthesis **(26a)** corresponds to one of the endings **(10)** of the first leaflet **(2)** and then to the nadir of the left coronary commissure, below the left coronary ostium **(32a).** Therefore, a proper placement of the artificial mechanical aortic valve prosthesis **(26a)** is possible. In this embodiment, the cardiac aorto-mitral prosthesis **(1)** may also be provided with a third leaflet **(17)** or a fourth leaflet **(18)** or both a third leaflet **(17)** and a fourth leaflet **(18)** on the other side of the mental partition **(4).**

The drawing FIG. 41 shows the process of construction of a cardiac aorto-mitral prosthesis (1) with four leaflets from a pericardial patch. The aorto-mitral prosthesis **(1)** with its first leaflet **(2)** and its second leaflet **(3)** is shaped to the desired form. Markings on the mental partition **(4)** and on the lengthwise mental partition **(16)** according to the judgement of the user are then applied. In this embodiment, the cardiac aorto-mitral prosthesis **(1)** has a mental partition **(4)** between the first **(2)** and the second leaflet **(3)** and a lengthwise mental partition **(16).** The combined third leaflet **(17)** and the fourth leaflet **(18)** are then shaped to the desired form and attached to the cardiac aorto-mitral prosthesis **(1)** with the first leaflet **(2)** and the second leaflet **(3).** In the most usual embodiment, the attachment of the third leaflet **(17)** or the fourth leaflet **(18)** or the third leaflet **(17)** and the fourth leaflet **(18)** is carried out on the other side of the mental partition **(4).**

The drawing FIG. 42 shows the next step in the process of construction of a cardiac aorto-mitral prosthesis with four leaflets from a pericardial patch. The first leaflet (2) and the second leaflet **(3)** of the cardiac aorto-mitral prosthesis **(1)** are sutured over the mental partition of the new aorto-mitral curtain **(4)** by the third leaflet sutures **(17c)** to the third leaflet pericardial patch **(17b)** and by the fourth leaflet sutures **(18c)** to the fourth leaflet pericardial patch **(18a).** Thus, a cardiac aorto-mitral prosthesis **(1)** with four leaflets is constructed. The third leaflet **(17)** is shown with the third leaflet pericardial patch **(17b)** and the fourth leaflet **(18)** is shown with the fourth leaflet pericardial patch **(18a).** The endings (**10**,**11**) of the first leaflet **(2)** and the endings **(14,15)** of the second leaflet **(3)** can be seen in the background. In the figure, the cardiac aorto-mitral prosthesis **(1)** is lifted by two pairs of tweezers **(29a,29b)** for a surgical operation.

The drawing FIG. 43 shows the cardiac aorto-mitral prosthesis **(1)** with four leaflets as seen from below. The third leaflet suturings **(17c)** of the third leaflet **(17)** to the first leaflet **(2)** and the second leaflet **(3)** can be seen. The first leaflet **(2)** and the second leaflet **(3)** with the third leaflet **(17)** for the covering of the aortic wall **(27a)** that is attached to the mental partition **(4)** of the cardiac aorto-mitral prosthesis **(1)** can be seen with the third leaflet sutures **(17c).** The cardiac aorto-mitral prosthesis **(1)** is lifted by a pair of tweezers **(29a).** The third leaflet **(17)** is attached to the cardiac aorto-mitral prosthesis **(1)** along the mental partition **(4)** between the first leaflet **(2)** and the second leaflet **(3)** by the third leaflet sutures **(17c).** A lengthwise mental partition **(16)** is present in this embodiment which is partially covered by the third leaflet sutures **(17c).** The lengthwise mental partition **(16)** may be formed for instance by a folding or a marking. The cardiac aorto-mitral prosthesis **(1)** is lifted by a pair of tweezers **(29a)** on the third leaflet **(17).**

The drawing FIG. 44 shows the cardiac aorto-mitral prosthesis **(1)** with the first leaflet **(2),** the second leaflet **(3),** the third leaflet **(17)** and the fourth leaflet **(18).** The third **(17)** and the fourth leaflet **(18)** are attached to each other along the mental partition **(4)** between the first leaflet **(2)** and the second leaflet **(3)** by the third leaflet sutures **(17c).** The cardiac aorto-mitral prosthesis **(1)** that is shown here is ready for use in a surgical operation for the replacement of the intervalvular fibrous body in the cardiac aorto-mitral curtain with the first leaflet **(2)** and the second leaflet **(3),** the replacement of the aorta-wall **(32)** with the third leaflet **(17)** and the replacement of the left atrial roof with the fourth leaflet **(18).**

The drawing FIG. 45 shows the cardiac aorto-mitral prosthesis **(1)** with an attached artificial biological mitral valve prosthesis **(30b)** and a third leaflet **(17)** and a fourth leaflet **(18).** The artificial biological mitral valve prosthesis **(30b)** has been sutured over the annular recess **(5)** by valve sutures **(39).** The third leaflet **(17)** for the replacement of the wall of the aorta **(32)** is visible in the background and the fourth leaflet **(18)** for the replacement of the left atrial roof **(28)** in the foreground. The cardiac aorto-mitral prosthesis **(1)** is lifted by a pair of tweezers **(29a)** on the third **(17)** and on the fourth leaflet **(18).** The artificial biological mitral valve prosthesis **(30b)** is stabilized by the valve supporting frame structure **(40)** to aid for the implantation during the surgical operation and will be removed during the surgical operation. The artificial biological mitral valve prosthesis **(30b)** is used for a replacement of the innate mitral valve **(21)** with the replacement of the aorto-mitral curtain **(25)** by the cardiac aorto-mitral prosthesis **(1),** the replacement of the aortic wall **(29a)** by the third leaflet **(17)** and of the left atrial roof **(28)** by the fourth leaflet **(18).** The cardiac aorto-mitral prosthesis **(1)** is lifted by a pair of tweezers **(29a)** on the third leaflet **(17)** and on the fourth leaflet **(18).**

The drawing FIG. 46 shows the cardiac aorto-mitral prosthesis **(1)** with an attached artificial biological mitral valve prosthesis **(30b)** seen from below, with the first leaflet **(2)** in the foreground and an attached third leaflet **(17).** The third leaflet **(17)** is visible in the background. The mental partition **(4)** and the lengthwise mental partition **(16)** have been marked. The attached artificial biological mitral valve prosthesis **(30b)** and the markings of the mental partitions **(4,16)** on the cardiac aorto-mitral prosthesis **(1)** allow rapid implantation and adequate positioning of the all commissures of the artificial biological mitral valve prosthesis **(30b)** in relation to the aorto-mitral curtain **(25),** thus shortening the surgical operation and improving the functional result of the operation. The cardiac aorto-mitral prosthesis **(1)** is lifted in the figure by a pair of tweezers **(29a).**

The drawing FIG. 47 shows the cardiac aorto-mitral prosthesis **(1)** with an attached artificial biological mitral valve prosthesis **(30b),** an attached artificial biological aortic valve prosthesis **(26b),** an attached third leaflet **(17)** and an attached fourth leaflet **(18)** in the foreground. In this embodiment of the cardiac aorto-mitral prosthesis **(1),** the aorto-mitral curtain **(25)** is replaced by the cardiac aorto-mitral prosthesis **(1)** and with it the innate mitral valve (21) and the innate aortic valve **(23)** are replaced. This embodiment is typically indicated when the innate mitral valve **(21)** and the innate aortic valve **(23)** have been damaged, for instance by inflammation. The artificial biological mitral valve prosthesis **(30b)** and the artificial biological aortic valve prosthesis **(26b)** are attached to the cardiac aorto-mitral prosthesis **(1)** by a valve suturing **(39).** Also, the valve supporting frame structure **(40)** of the artificial biological mitral valve prosthesis **(30b)** which aids during the implantation and which is removed during the surgical operation is shown. In the figure, the cardiac aorto-mitral prosthesis **(1)** is lifted by a pair of tweezers **(29a)** on the third **(17)** and on the fourth leaflet **(18).**

The drawing FIG. 48 shows the cardiac aorto-mitral prosthesis **(1)** with an attached artificial biological mitral valve prosthesis **(30b),** an attached artificial biological aortic valve prosthesis **(26b),** an attached third leaflet **(17)** and an attached fourth leaflet **(18).** With this embodiment of the cardiac aorto-mitral prosthesis **(1),** the aorto-mitral curtain **(25)** can be replaced, the damaged mitral valve can be replaced, the damaged aortic valve can be replaced, the aortic wall **(27a)** of the aorta **(27)** can be replaced, and the left atrial roof **(28)** can be replaced.

The drawing FIG. 49 shows the cardiac aorto-mitral prosthesis **(1)** with the first leaflet **(2)** and the second leaflet **(3).** The first leaflet **(2)** is attached to an artificial mitral valve prosthesis **(30).** The second leaflet **(3)** is attached to an artificial aortic valve prosthesis **(26).** The mental partition **(4)** and the lengthwise mental partition **(16)** are shown. In this embodiment, the distance **(16b)** between the first leaflet recess **(5)** and the mental partition is 1 mm. The distance **(16a)** between the mental partition and the second leaflet recess **(6)** is 1 mm. Therefore the distance between the first leaflet recess **(5)** and the second leaflet recess **(6)** is 2 mm. The attached artificial mitral valve prosthesis **(30)** and the attached artificial aortic valve prosthesis **(26)** allow rapid implantation and adequate positioning of both valves **(26,30)** in relation to the aorto-mitral curtain **(25),** thus shortening the surgical operation and improving the functional result of the operation.

The drawing FIG. 50 shows the cardiac aorto-mitral prosthesis **(1)** with four leaflets, an attached artificial mechanical mitral valve prosthesis **(30a),** an attached artificial mechanical aortic valve prosthesis **(26a),** and attached third leaflet **(17)** and an attached fourth leaflet **(18).** The attached artificial mechanical mitral valve prosthesis **(30a)** and the attached mechanical artificial aortic valve prosthesis **(26a)** allow rapid implantation and adequate positioning of both valves (**26a**,**30a**) in relation to the aorto-mitral curtain **(25),** thus shortening the surgical procedure and improving the functional result of the operation. The cardiac aorto-mitral prosthesis **(1)** is lifted by a pair of tweezers **(29a)** on the third **(17)** and on the fourth leaflet **(18).**

The drawing FIG. 51 shows the cardiac aorto-mitral prosthesis **(1)** with four leaflets, an attached artificial mechanical mitral valve prosthesis **(30a),** an attached artificial mechanical aortic valve prosthesis **(26a),** an attached third leaflet **(17)** and an attached fourth leaflet **(18)** from another perspective.

The drawing FIG. 52 shows the cardiac aorto-mitral prosthesis **(1)** with an attached artificial mechanical mitral valve prosthesis **(30a),** an attached artificial mechanical aortic valve prosthesis **(26a),** and an attached third leaflet **(17)** and an attached fourth leaflet **(18).** The attached third leaflet **(17)** and the attached fourth leaflet **(18)** are visible in the background and are sutured to the mental partition **(4)** on the other side. The lengthwise mental partition **(16)** is also seen. The artificial mechanical mitral valve prosthesis **(30a)** and the artificial mechanical aortic valve prosthesis **(26a)** are attached to the cardiac aorto-mitral prosthesis **(1)** by sutures. The artificial mechanical aortic valve prosthesis **(26a)** has been attached to the first leaflet recess **(5)** and the artificial mechanical mitral valve prosthesis **(30a)** has been attached to the second leaflet recess **(6).**

The drawing FIG. 53 shows the cardiac aorto-mitral prosthesis **(1)** being implanted into the heart **(20)** during a surgical operation, when staying on the right side of the patient and looking through the aortic wall incision **(27b)** towards the innate aortic valve **(23)** and aorto-mitral curtain **(25).** To the cardiac aorto-mitral prosthesis **(1),** a third leaflet **(17),** a fourth leaflet **(18)** and an artificial biological aortic valve prosthesis **(26b)** is attached. The third leaflet **(17)** is replacing and closing the aortic wall incision **(27b)** in the aortic wall **(27a)** by suturing. The third leaflet sutures **(17c)** which suture the third leaflet pericardial patch **(17b)** and the fourth leaflet pericardial patch **(18a)** to each other are visible. The fourth leaflet pericardial patch **(18a)** and the aortic root **(23b)** are also visible. The artificial biological aortic valve prosthesis **(26b)** has been positioned in the second leaflet recess **(6)** of the second leaflet **(3)** and will be sutured to the aortic valve annulus **(23a)** after the removal of the valve supporting frame structure **(40).**

The drawing FIG. 54 shows the last step of the implantation of the cardiac aorto-mitral prosthesis **(1)** with four leaflets before the closure of the aortic wall incision **(27b)** of the aortic wall **(27a)** and the ending the surgical operation. The valve supporting frame structure **(40)** of an artificial biological aortic valve prosthesis **(26b)** has been removed and the artificial biological aortic valve prosthesis **(26b)** is sutured to the aortic valve annulus **(23a)** by valve suturing **(39).** The third leaflet **(17)** has replaced and closed the aortic wall incision **(27b)** by direct third leaflet-aortic wall suture **(17d).** The left atrial roof **(28)** has been closed with the fourth leaflet pericardial patch **(18)** of the cardiac aorto-mitral prosthesis by suturing **(18c).**

The drawing FIG. 55 shows the cardiac aorto-mitral prosthesis **(1)** with four leaflets as seen after opening the left ventricle at the end of the surgical operation to the human heart **(20)** in an in vitro experiment. The cardiac aorto-mitral prosthesis **(1)** has been provided with an artificial biological aortic valve prosthesis **(26b),** an artificial biological mitral valve prosthesis **(30b)** and a third leaflet **(17).** The left fibrous trigone **(25a),** the right fibrous trigone **(25b)** and the intervalvular fibrous body have been replaced by the first leaflet **(2)** and the second leaflet **(3)** of the cardiac aorto-mitral prosthesis **(1).** The mental partition **(4)** and the suture line **(17c)** between the third leaflet **(17)** and fourth leaflet **(18)** has been fitted into the aorto-mitral angle α **(35)** and can be seen only in part in this figure. The anterior cusp center of the innate aortic valve **(23)** has been aligned by the ending marking **(14a)** of the second leaflet **(3)** with the artificial biological mitral valve prosthesis **(30b).** The commissure between non-coronary cusp and right coronary cusp of the artificial biological aortic valve prosthesis **(26b)** has been fitted into the aorto-mitral angle α **(35)** below the mitral valve annulus **(21a)** with the artificial biological mitral valve prosthesis **(30b)** and is situated at the end of the coaptation line of the aortic valve cusps. The aorto-mitral angle α **(35)** cannot be seen in this figure.

The drawing FIG. 56 shows the cardiac aorto-mitral prosthesis **(1)** with two leaflets and an attached artificial biological aortic valve prosthesis **(26b).** The cardiac aorto-mitral prosthesis **(1)** has been implanted and is seen after opening the left ventricle at the end of the surgical operation in an in vitro experiment. The cardiac aorto-mitral prosthesis **(1)** has been turned upside-down in order to face the aorto-mitral angle α **(35).** After turning the cardiac aorto-mitral prosthesis **(1)** upside-down, the first leaflet **(2)** and second leaflet **(3)** of the cardiac aorto-mitral prosthesis **(1)** are exposed and show the first leaflet ending **(10)** and the second leaflet ending (14) to replace the left fibrous trigone **(25a)** of the human heart **(20),** and the first leaflet extension **(11)** and the second leaflet ending **(15)** to replace the right fibrous trigone **(25b)** of the human heart **(20).** The mental partition **(4)** the between the first leaflet **(2)** and the second leaflet **(3)** is shown. It has been fitted into the aorto-mitral angle **(35).** The anterior cusp center of the innate aortic valve **(23)** can be seen along the central part **(25c)** of the cardiac aorto-mitral prosthesis **(1)** The position of the artificial biological aortic valve prosthesis **(26b)** may be aligned along the lengthwise mental partition **(16).** The commissure between the non-coronary cusp and right coronary cusp of the artificial biological aortic valve prosthesis **(26b)** has been fitted into the aorto-mitral angle **(35)** below the mitral valve annulus **(21a)** to which the artificial biological mitral valve prosthesis **(30b)** has been sutured **(39).** By this embodiment, the capacity of the present invention to replace the fibrous trigons **(25a,25b),** to reduce the tension on the valve sutures **(39),** to re-establish the innate anatomic aorto-mitral angle **(35)** and to allow optimal positioning of the cardiac aorto-mitral prosthesis **(1)** is shown.

### List of reference numerals

- 1: Cardiac aorto-mitral prosthesis
- 2: First leaflet
- 3: Second leaflet
- 4: Mental partition
- 5: First leaflet recess
- 6: Second leaflet recess
- 7: Larger recess
- 8: Outer edge
- 9: First leaflet ending
- 10: First leaflet ending
- 11: First leaflet extension
- 11a: Ending marking
- 12: Ending of second leaflet
- 13: Ending of second leaflet
- 14: Second leaflet ending
- 14a: Ending marking
- 15: Second leaflet ending
- 15a: Ending marking
- 16: Lengthwise mental partition
- 17: Third leaflet
- 17a: Third leaflet straight cutting
- 17b: Third leaflet pericardial patch
- 17c: Third leaflet sutures
- 17d: Direct third leaflet-aortic wall suture
- 18: Fourth leaflet
- 18a: Fourth leaflet pericardial patch
- 18b: Fourth leaflet straight cutting
- 18c: Fourth leaflet sutures
- 19: Recess extension
- 19a: Recess ending
- 19b: Recess extension boundary
- 20: Human heart
- 21: Mitral valve
- 21a: Mitral valve annulus
- 21b: Anterior cusp of innate mitral valve
- 21c: Posterior cusp of the innate mitral valve
- 21d: Annuloplasty band
- 22: Tricuspid valve
- 22a: Tricuspid valve annulus
- 23: Aortic valve
- 23a: Aortic valve annulus
- 23b: Aortic root
- 24: Pulmonary valve
- 25: Aorto-mitral curtain
- 25a: Left trigone
- 25b: Right trigone
- 25c: Central part of the aorto-mitral curtain
- 26: Artificial aortic valve prosthesis
- 26a: Artificial mechanical aortic valve prosthesis
- 26b: Artificial biological aortic valve prosthesis
- 27: Ascending aorta
- 27a: Aortic wall
- 27b: Aortic wall incision
- 27c: Tissue around the aorta
- 28: Left atrial roof
- 29a: Pair of tweezers
- 29b: Another pair of tweezers
- 30: Artificial mitral valve prosthesis
- 30a: Artificial mechanical mitral valve prosthesis
- 30b: Artificial biological mitral valve prosthesis
- 31: Annular sutures
- 32a: Left coronary ostium
- 32b: Right coronary ostium
- 33: Surgical instruments
- 34: Aorta tube prosthesis with valve
- 35a: Aorto-mitral angle α
- 35b: Stretching angle β
- 36: Fingers
- 37: Left ventricular outflow tract
- 38: Pair of clamps
- 39: Valve sutures
- 40: Valve supporting frame structure

## Claims

1. A cardiac aorto-mitral prosthesis (1) for the human heart (20), consisting of a pericardial patch, forming a template of the aorto-mitral curtain (25), comprising
• two leaflets (2,3) separated by a mental partition (4) that is a central line and separates the cardiac aorto-mitral prosthesis (1) into a first leaflet (2) and a second leaflet (3) for a placement of the cardiac aorto-mitral prosthesis between the aortic valve and the mitral valve during a surgical operation to the human heart, and
• one first leaflet (2) having a first leaflet recess (5) which serves for the inclusion of the aortic valve annulus (23a), and the other second leaflet (3) having a second leaflet recess (6) which serves for the inclusion of the mitral valve annulus (21a),
**characterized in that**
• the cardiac aorto-mitral prosthesis (1) has over both leaflets (2,3) one larger recess (7) for the support to the tricuspid valve annulus (22a), and
• on the opposite side of the larger recess (7) over both leaflets (2,3) an outer edge (8) which forms a support to the frame of the inner layer of the atrio-ventricular junction of the human heart (20).

2. The cardiac aorto-mitral prosthesis (1) according to claim 1, **characterized in that** the mental partition (4) is marked by a visible line or by a folding.

3. The cardiac aorto-mitral prosthesis (1) according to claim 2, **characterized in that** markings are present on the aorto-mitral prosthesis (1) that mark the positions of the commissures of the aortic valve or of the mitral valve (21).

4. The cardiac aorto-mitral prosthesis (1) according to one of claims 1 to 3, **characterized in that** another third leaflet (17) is attached to the prosthesis (1) along the mental partition (4) for a covering of the aortic wall (27a) of the aorta (27).

5. The cardiac aorto-mitral prosthesis (1) according to one of claims 1 to 4, **characterized in that** another fourth leaflet (18) is attached to the prosthesis along the mental partition (4) for a covering of the left atrial roof.

6. The cardiac aorto-mitral prosthesis (1) according to one of claims 1 to 5, **characterized in that** the cardiac aorto-mitral prosthesis (1) has a first leaflet extension (11) that extends the ending (9) of the right aortic annular part that includes the aortic valve (23) and supports the tricuspid valve annulus (22a).

7. The cardiac aorto-mitral prosthesis (1) according to claim 6, **characterized in that** the first leaflet extension (11) is marked by a folding (11a) or by a visible line in separation from an ending (9) of the first leaflet (2) that includes the aortic valve (23) and supports the tricuspid valve annulus (22a).

8. The cardiac aorto-mitral prosthesis (1) according to one of claims 1 to 7, **characterized in that** the cardiac aorto-mitral prosthesis (1) has a recess extension (19) over the recess ending (19a) of the second leaflet (3) in separation from the unextended cardiac aorto-mitral prosthesis (1) of the pericardial patch of the cardiac aorto-mitral prosthesis (1) over the anterior cusp of the mitral valve annulus (21a).

9. The cardiac aorto-mitral prosthesis (1) according to claim 8, **characterized in that** the recess extension (19) is marked by a visible line over the recess ending (19a) in separation from the unextended cardiac aorto-mitral prosthesis (1).

10. The cardiac aorto-mitral prosthesis (1) according to one of claims 1 to 9, **characterized in that** an artificial mitral valve prosthesis (30) is attached to the cardiac aorto-mitral prosthesis (1).

11. The cardiac aorto-mitral prosthesis (1) according to one of claims 1 to 10, **characterized in that** an artificial aortic valve prosthesis (26) is attached to the cardiac aorto-mitral prosthesis (1).

12. The cardiac aorto-mitral prosthesis (1) according to one of claims 1 to 11, **characterized in that** the cardiac aorto-mitral prosthesis (1) is made of a pericardial patch that is made from bovine pericardium or Dacron-fabric or Teflon fabric.

13. A process for the production of a cardiac aorto-mitral prosthesis (1) according to one of claims 1 to 12, wherein a pericardial patch is taken and cut into two leaflets (2,3) separated by a mental partition (4) of the cardiac aorto-mitral prosthesis (1) that is a central line and that separates the cardiac aorto-mitral prosthesis (1) into the first leaflet (2) and second leaflet (3) as two annular parts (2,3) between the aortic valve annulus (23a) and the mitral valve annulus (21a) of the human heart, and
• into one first leaflet (2) a first leaflet recess (5) is cut which serves for the inclusion of the aortic valve annulus (23a), and
• into the other second leaflet (3) a second leaflet recess (6) is cut which serves for the inclusion of the mitral valve annulus (21a), **characterized in that**
• into the prosthesis (1) one larger recess (7) is cut over both leaflets (2,3) for the support of the tricuspid valve annulus (22a), and
• into the opposite side of the larger recess (7) an outer edge (8) is cut which forms a support to the frame of the inner layer of the atrio-ventricular junction of the human heart (20).

14. The process for the production of a cardiac aorto-mitral prosthesis (1) according to claim 13, **characterized in that** a further third leaflet (17) is attached to the cardiac aorto-mitral prosthesis (1) for a covering of the aortic wall (27a) of the aorta (27) or a further fourth leaflet (18) as a further leaflet for a covering of the left atrial roof or a third (17) and a fourth leaflet (18) as further leaflets is or are attached to the cardiac aorto-mitral prosthesis (1).

15. The process for the production of a cardiac aorto-mitral prosthesis (1) according to claim 13 or claim 14, **characterized in that** a recess extension (19) is left in the second leaflet recess (6) of the pericardial patch over the mitral valve annulus (21a) in the cutting of the second leaflet recess (6) for the mitral valve annulus (21a).

## Patentansprüche

1. Aortomitrale Herzprothese (1) für das menschliche Herz (20), bestehend aus einem Perikardpatch, der eine Schablone des aortomitralen Vorhangs (25) bildet, umfassend
• zwei Segel (2, 3), die durch eine mentale Unterteilung (4) getrennt sind, die eine Mittellinie ist und die aortomitrale Herzprothese (1), zur Platzierung der aortomitralen Herzprothese während eines chirurgischen Eingriffs am menschlichen Herzen zwischen der Aortenklappe und der Mitralklappe, in ein erstes Segel (2) und ein zweites Segel (3) trennt, und
• wobei ein erstes Segel (2) eine erste Segelaussparung (5) aufweist, die zur Aufnahme des Aortenklappenanulus (23a) dient, und das andere, zweite Segel (3) eine zweite Segelaussparung (6) aufweist, die zur Aufnahme des Mitralklappenanulus (21a) dient,
**dadurch gekennzeichnet, dass**
• die aortomitrale Herzprothese (1) über beide Segel (2, 3) eine größere Aussparung (7) zur Stützung des Trikuspidalklappenanulus (22a) aufweist, und
• auf der entgegengesetzten Seite der größeren Aussparung (7) über beide Segel (2, 3) einen Außenrand (8) aufweist, der eine Stütze für das Gerüst der inneren Schicht des atrioventrikulären Übergangs des menschlichen Herzens (20) bildet.

2. Aortomitrale Herzprothese (1) nach Anspruch 1, **dadurch gekennzeichnet, dass** die mentale Unterteilung (4) durch eine sichtbare Linie oder durch eine Faltung markiert ist.

3. Aortomitrale Herzprothese (1) nach Anspruch 2, **dadurch gekennzeichnet, dass** Markierungen an der aortomitralen Prothese (1) vorhanden sind, die die Positionen der Kommissuren der Aortenklappe oder der Mitralklappe (21) markieren.

4. Aortomitrale Herzprothese (1) nach einem der Ansprüche 1 bis 3, **dadurch gekennzeichnet, dass** ein anderes, drittes Segel (17) zur Abdeckung der Aortenwand (27a) der Aorta (27) entlang der mentalen Unterteilung (4) an der Prothese (1) angebracht ist.

5. Aortomitrale Herzprothese (1) nach einem der Ansprüche 1 bis 4, **dadurch gekennzeichnet, dass** ein anderes, viertes Segel (18) zur Abdeckung des Dachs des linken Vorhofs entlang der mentalen Unterteilung (4) an der Prothese angebracht ist.

6. Aortomitrale Herzprothese (1) nach einem der Ansprüche 1 bis 5, **dadurch gekennzeichnet, dass** die aortomitrale Herzprothese (1) eine erste Segelerweiterung (11) aufweist, die das Ende (9) des rechten kranzförmigen Aortenteils erweitert, das die Aortenklappe (23) umfasst und den Trikuspidalklappenanulus (22a) stützt.

7. Aortomitrale Herzprothese (1) nach Anspruch 6, **dadurch gekennzeichnet, dass** die erste Segelerweiterung (11) durch eine Faltung (11a) oder durch eine sichtbare Linie als Trennung von einem Ende (9) des ersten Segels (2) markiert ist, das die Aortenklappe (23) umfasst und den Trikuspidalklappenanulus (22a) stützt.

8. Aortomitrale Herzprothese (1) nach einem der Ansprüche 1 bis 7, **dadurch gekennzeichnet, dass** die aortomitrale Herzprothese (1) eine Aussparungserweiterung (19) über das Aussparungsende (19a) des zweiten Segels (3) als Trennung von der nicht erweiterten aortomitralen Herzprothese (1) des Perikardpatches der aortomitralen Herzprothese (1) über den vorderen Zipfel des Mitralklappenanulus (21a) aufweist.

9. Aortomitrale Herzprothese (1) nach Anspruch 8, **dadurch gekennzeichnet, dass** die Aussparungserweiterung (19) durch eine sichtbare Linie über das Aussparungsende (19a) als Trennung von der nicht erweiterten aortomitralen Herzprothese (1) markiert ist.

10. Aortomitrale Herzprothese (1) nach einem der Ansprüche 1 bis 9, **dadurch gekennzeichnet, dass** eine künstliche Mitralklappenprothese (30) an der aortomitralen Herzprothese (1) angebracht ist.

11. Aortomitrale Herzprothese (1) nach einem der Ansprüche 1 bis 10, **dadurch gekennzeichnet, dass** eine künstliche Aortenklappenprothese (26) an der aortomitralen Herzprothese (1) angebracht ist.

12. Aortomitrale Herzprothese (1) nach einem der Ansprüche 1 bis 11, **dadurch gekennzeichnet, dass** die aortomitrale Herzprothese (1) aus einem Perikardpatch hergestellt ist, der aus bovinem Perikard oder Dacron-Gewebe oder Teflon-Gewebe hergestellt ist.

13. Verfahren zur Herstellung einer aortomitralen Herzprothese (1) nach einem der Ansprüche 1 bis 12, wobei ein Perikardpatch genommen und in zwei Segel (2, 3) geschnitten wird, die durch eine mentale Unterteilung (4) der aortomitralen Herzprothese (1) getrennt sind, die eine Mittellinie ist und die aortomitrale Herzprothese (1) in das erste Segel (2) und das zweite Segel (3) als zwei kranzförmige Teile (2, 3) zwischen dem Aortenklappenanulus (23a) und dem Mitralklappenanulus (21a) des menschlichen Herzens teilt, und
• wobei in ein erstes Segel (2) eine erste Segelaussparung (5) geschnitten wird, die zur Aufnahme des Aortenklappenanulus (23a) dient, und
• wobei in das andere, zweite Segel (3) eine zweite Segelaussparung (6) geschnitten wird, die zur Aufnahme des Mitralklappenanulus (21a) dient,
**dadurch gekennzeichnet, dass**
• über beide Segel (2, 3) eine größere Aussparung (7) zur Stützung des Trikuspidalklappenanulus (22a) in die Prothese (1) geschnitten wird, und
• in die entgegengesetzte Seite der größeren Aussparung (7) ein Außenrand (8) geschnitten wird, der eine Stütze für das Gerüst der inneren Schicht des atrioventrikulären Übergangs des menschlichen Herzens (20) bildet.

14. Verfahren zur Herstellung einer aortomitralen Herzprothese (1) nach Anspruch 13, **dadurch gekennzeichnet, dass** ein weiteres, drittes Segel (17) zur Abdeckung der Aortenwand (27a) der Aorta (27) an der aortomitralen Herzprothese (1) angebracht wird, oder ein weiteres, viertes Segel (18) als ein weiteres Segel zur Abdeckung des Dachs des linken Vorhofs, oder ein drittes (17) und ein viertes (18) Segel als weitere Segel an der aortomitralen Herzprothese (1) angebracht wird oder werden.

15. Verfahren zur Herstellung einer aortomitralen Herzprothese (1) nach Anspruch 13 oder Anspruch 14, **dadurch gekennzeichnet, dass** beim Schneiden der zweiten Segelaussparung (6) für den Mitralklappenanulus (21a) eine Aussparungserweiterung (19) in der zweiten Segelaussparung (6) des Perikardpatches über den Mitralklappenanulus (21a) zurückbleibt.

## Revendications

1. Prothèse (1) cardiaque aorto-mitrale pour le cœur humain (20), constituée d'un pansement péricardique, formant un modèle du rideau aorto-mitral (25), comportant
• deux feuillets valvulaires (2, 3) séparés par une séparation mentale (4) qui est une ligne centrale et sépare la prothèse (1) cardiaque aorto-mitrale en un premier feuillet valvulaire (2) et un deuxième feuillet valvulaire (3) pour un placement de la prothèse cardiaque aorto-mitrale entre la valvule aortique et la valvule mitrale pendant une opération chirurgicale du cœur humain et
• un premier feuillet valvulaire (2) ayant un premier évidement (5) de feuillet valvulaire qui sert pour l'inclusion de l'anneau (23a) de valvule aortique et l'autre deuxième feuillet valvulaire (3) ayant un second évidement (6) de feuillet valvulaire qui sert pour l'inclusion de l'anneau (21a) de valvule mitrale,
**caractérisée en ce que**
• la prothèse (1) cardiaque aorto-mitrale a, sur les deux feuillets valvulaires (2, 3), un évidement (7) plus grand pour le soutien de l'anneau (22a) de valvule tricuspide et
• sur le côté opposé de l'évidement (7) plus grand sur les deux feuillets valvulaires (2, 3), un bord extérieur (8) qui forme un soutien pour le cadre de la couche intérieure de la jonction atrio-ventriculaire du cœur humain (20).

2. Prothèse (1) cardiaque aorto-mitrale selon la revendication 1, **caractérisée en ce que** la séparation mentale (4) est marquée par une ligne visible ou par un pliage.

3. Prothèse (1) cardiaque aorto-mitrale selon la revendication 2, **caractérisée en ce que** des marquages sont présents sur la prothèse (1) aorto-mitrale, lesquels marquent les positions des commissures de la valvule aortique ou de la valvule mitrale (21).

4. Prothèse (1) cardiaque aorto-mitrale selon l'une des revendications 1 à 3, **caractérisée en ce qu'**un autre troisième feuillet valvulaire (17) est fixé à la prothèse (1) le long de la séparation mentale (4) pour une couverture de la paroi aortique (27a) de l'aorte (27).

5. Prothèse (1) cardiaque aorto-mitrale selon l'une des revendications 1 à 4, **caractérisée en ce qu'**un autre quatrième feuillet valvulaire (18) est fixé à la prothèse le long de la séparation mentale (4) pour une couverture du plafond atrial gauche.

6. Prothèse (1) cardiaque aorto-mitrale selon l'une des revendications 1 à 5, **caractérisée en ce que** la prothèse (1) cardiaque aorto-mitrale a une première extension de feuillet valvulaire (11) qui étend la terminaison (9) de la partie annulaire aortique droite qui inclut la valvule aortique (23) et soutient l'anneau (22a) de valvule tricuspide.

7. Prothèse (1) cardiaque aorto-mitrale selon la revendication 6, **caractérisée en ce que** la première extension (11) de feuillet valvulaire est marquée par un pliage (11a) ou par une ligne visible de séparation par rapport à une terminaison (9) du premier feuillet valvulaire (2) qui inclut la valvule aortique (23) et soutient l'anneau (22a) de valvule tricuspide.

8. Prothèse (1) cardiaque aorto-mitrale selon l'une des revendications 1 à 7, **caractérisée en ce que** la prothèse (1) cardiaque aorto-mitrale a une extension (19) d'évidement sur la terminaison (19a) d'évidement du deuxième feuillet valvulaire (3) en séparation par rapport à la prothèse (1) cardiaque aorto-mitrale non étendue du pansement péricardique de la prothèse (1) cardiaque aorto-mitrale sur la cuspide antérieure de l'anneau (21a) de valvule mitrale.

9. Prothèse (1) cardiaque aorto-mitrale selon la revendication 8, **caractérisée en ce que** l'extension (19) d'évidement est marquée par une ligne visible sur la terminaison (19a) d'évidement en séparation par rapport à la prothèse (1) cardiaque aorto-mitrale non étendue.

10. Prothèse (1) cardiaque aorto-mitrale selon l'une des revendications 1 à 9, **caractérisée en ce qu'**une prothèse (30) de valvule mitrale artificielle est fixée à la prothèse (1) cardiaque aorto-mitrale.

11. Prothèse (1) cardiaque aorto-mitrale selon l'une des revendications 1 à 10, **caractérisée en ce qu'**une prothèse (26) de valvule aortique artificielle est fixée à la prothèse (1) cardiaque aorto-mitrale.

12. Prothèse (1) cardiaque aorto-mitrale selon l'une des revendications 1 à 11, **caractérisée en ce que** la prothèse (1) cardiaque aorto-mitrale est constituée d'un pansement péricardique qui est constitué d'un péricarde bovin ou d'un tissu Dacron ou d'un tissu Teflon.

13. Processus pour la production d'une prothèse (1) cardiaque aorto-mitrale selon l'une des revendications 1 à 12, dans lequel un pansement péricardique est pris et coupé en deux feuillets valvulaires (2, 3) séparés par une séparation mentale (4) de la prothèse (1) cardiaque aorto-mitrale qui est une ligne centrale et qui sépare la prothèse (1) cardiaque aorto-mitrale en le premier feuillet valvulaire (2) et le deuxième feuillet valvulaire (3) en tant que deux parties annulaires (2, 3) entre l'anneau (23a) de valvule aortique et l'anneau (21a) de valvule mitrale du cœur humain et
• dans un premier feuillet valvulaire (2), un premier évidement (5) de feuillet valvulaire est découpé, lequel sert pour l'inclusion de l'anneau (23a) de valvule aortique et
• dans l'autre deuxième feuillet valvulaire (3), un second évidement (6) de feuillet valvulaire est découpé, lequel sert pour l'inclusion de l'anneau (21a) de valvule mitrale,
**caractérisé en ce que**
• dans la prothèse (1), un évidement (7) plus grand est découpé sur les deux feuillets valvulaires (2, 3) pour le soutien de l'anneau (22a) de valvule tricuspide et
• dans le côté opposé de l'évidement (7) plus grand, un bord extérieur (8) est découpé, lequel forme un soutien du cadre de la couche intérieure de la jonction atrio-ventriculaire du cœur humain (20).

14. Processus pour la production d'une prothèse (1) cardiaque aorto-mitrale selon la revendication 13, **caractérisé en ce qu'**un autre troisième feuillet valvulaire (17) est fixé à la prothèse (1) cardiaque aorto-mitrale pour une couverture de la paroi aortique (27a) de l'aorte (27) ou **en ce qu'**un autre quatrième feuillet valvulaire (18) en tant qu'autre feuillet valvulaire pour une couverture du plafond atrial gauche ou **en ce qu'**un troisième (17) et un quatrième feuillet valvulaire (18) en tant qu'autres feuillets valvulaires est ou sont fixés à la prothèse (1) cardiaque aorto-mitrale.

15. Processus pour la production d'une prothèse (1) cardiaque aorto-mitrale selon la revendication 13 ou la revendication 14, **caractérisé en ce qu'**une extension (19) d'évidement est laissée dans le second évidement (6) de feuillet valvulaire du pansement péricardique sur l'anneau (21a) de valvule mitrale dans la découpe du second évidement (6) de feuillet valvulaire pour l'anneau (21a) de valvule mitrale.
